# EUROPÄISCHE PATENTSCHRIFT

(11) **EP 3 511 404 B1**
(45) Veröffentlichungstag und Bekanntmachung des Hinweises auf die Patenterteilung: **18.12.2024**
(21) Anmeldenummer: 19158709.6
(22) Anmeldetag: 18.06.2015
(51) Int. Cl.: C11D 3/386

(54) **FLÜSSIGES REINIGUNGSMITTEL ENTHALTEND FLÜSSIGE UND FESTE ENZYMFORMULIERUNGEN**
LIQUID CLEANING COMPOSITION CONTAINING LIQUID AND SOLID ENZYME FORMULATIONS
DÉTERGENT LIQUIDE CONTENANT DES FORMULATIONS ENZYMATIQUES LIQUIDES ET SOLIDES

(30) Priorität: 30.06.2014 DE 102014212643
(43) Veröffentlichungstag der Anmeldung: 17.07.2019
(62) Teilanmeldung aus: 15730146.6
(73) Patentinhaber: Henkel AG & Co. KGaA, 40589 Düsseldorf (DE)
(72) Erfinder: Mussmann, Nina, 47877 Willich (DE); Eiting, Thomas, 40589 Düsseldorf (DE); Wrubbel, Noelle, 40591 Düsseldorf (DE); Bastigkeit, Thorsten, 42279 Wuppertal (DE)

(56) Entgegenhaltungen:
- EP-A1- 2 100 949
- EP-A2- 1 921 148
- WO-A1-2010/090915
- WO-A1-2011/036263
- DE-A1- 19 918 457
- GB-A- 2 392 449
- US-A1- 2014 179 585
- US-B1- 6 287 841

## Beschreibung

Die vorliegende Anmeldung richtet sich auf ein flüssiges Reinigungsmittel, bevorzugt ein Geschirrspülmittel, vorzugsweise ein maschinelles Geschirrspülmittel, das mindestens eine feste und mindestens eine flüssige Enzymformulierung enthält, sowie die Verwendung eines solchen Reinigungsmittels.

Das wichtigste Kriterium beim Reinigen von harten Oberflächen, insbesondere beim maschinellen Geschirrspülen, ist die Reinigungsleistung an verschiedensten Anschmutzungen, welche insbesondere in Form von Lebensmittelresten eingebracht werden. Auch wenn die Reinigungsleistung der heutzutage eingesetzten Geschirrspülmittel generell hoch ist, stellt sich, auch aufgrund des generellen Trends beim maschinellen Geschirrspülen vermehrt Niedrigtemperatur-Programme zu verwenden, allerdings das Problem, dass viele der üblichen maschinellen Geschirrspülmittel bei hartnäckigen Anschmutzungen eine unzureichende Reinigungsleistung aufweisen. Eine solche unzureichende Reinigungsleistung und die damit einhergehende unzureichende Reinigung des Geschirrs führen beim Verbraucher zu Unzufriedenheit und dazu, dass solche Anschmutzungen vom Verbraucher vorbehandelt werden, was wiederum den Wasser- und Energieverbrauch erhöht. Daher besteht ein allgemeiner Bedarf nach maschinellen Geschirrspülmitteln, die auch an hartnäckigen Anschmutzungen noch eine gute Reinigungsleistung aufweisen.

Reinigungsmittel für harte Oberflächen, wie beispielsweise Geschirrspülmittel, stehen dem Verbraucher in einer Vielzahl von Angebotsformen zur Verfügung. Neben den traditionellen festen Mitteln gewinnen in letzter Zeit zunehmend fließfähige und insbesondere flüssige bis gelförmige Reinigungsmittel an Bedeutung. Der Verbraucher schätzt vor allem die schnelle Löslichkeit und die damit einhergehende schnelle Verfügbarkeit der Inhaltsstoffe in der Reinigungsflotte insbesondere auch in Kurzgeschirrspülprogrammen und bei niedrigen Temperaturen. Aus z.B. GB 2392449 A, DE 19918457 A1, EP 2100949 A1 und WO 2010/090915 A1, WO 2011/036263A1, US 6287841 B1, US 2014/179585 A1 und EP 1921148 A2 sind enzymhaltige, insbesondere protease- und/oder amylasehaltige, Wasch- und/oder Reinigungsmittel bekannt.

Dabei nimmt die Bedeutung von konzentrierten Zusammensetzungen, in denen insbesondere der Wassergehalt gegenüber herkömmlichen Zusammensetzungen vermindert ist, zu. Für den Verbraucher sind somit Zusammensetzungen, deren Wassergehalt möglichst gering ist, beispielsweise kleiner 25 Gew.-%, besonders wünschenswert.

Ferner haben sich die Verbraucher an ein bequemes Dosieren von vorportionierten maschinellen Geschirrspülmitteln gewöhnt und nutzen diese Produkte bisher vor allem in Form von Tabletten. Um ein flüssiges Geschirrspülmittel, das die oben erwähnten Vorteile gegenüber festen Zusammensetzungen bietet, in eine vorportionierte Angebotsform zu bringen, ist die Verwendung von kaltwasserlöslichen Folien in der Form von Beuteln üblich. Der Formelentwicklung sind damit jedoch Grenzen gesetzt, da nur eine begrenzte Menge Wasser in das Produkt eingearbeitet werden kann. Eine Überschreitung der tolerierbaren Wassermenge führt zu einem vorzeitigen Auflösen der umhüllenden wasserlöslichen Folie. Um eine gute Lagerstabilität dieser wasserlöslichen Behälter zu gewährleisten, sind ebenfalls Wassergehalte von weniger als 25 Gew.-% wünschenswert.

Des Weiteren sind flüssige Formulierungen für den Verbraucher optisch besonders ansprechend, wenn sie stabil suspendierte, feste ggf. auch farbige Bestandteile enthalten. Wenn diese festen Bestandteile reinigungsaktive Substanzen enthalten und diese als besonders leistungsstark ausgelobt werden, vermittelt das dem Konsumenten den Eindruck, dass ein derartiges Produkt leistungsstärker ist als ein Produkt ohne diese festen Bestandteile.

Die Aufgabe der vorliegenden Erfindung bestand daher darin, ein flüssiges Reinigungsmittel, bevorzugt ein Geschirrspülmittel, vorzugsweise ein maschinelles Geschirrspülmittel, zur Verfügung zu stellen, das eine gesteigerte Reinigungsleistung aufweist, einen geringen Wassergehalt aufweist und optisch ansprechend ist.

Überraschenderweise wurde nun festgestellt, dass die Verwendung einer Kombination aus flüssigen Enzymformulierungen und festen Enzymgranulaten zu einer gegenüber der alleinigen Verwendung von entweder flüssiger oder fester Enzymformulierung verbesserten Leistung von flüssigen Geschirrspülmitteln führt. Des Weiteren bietet die Verwendung fester Enzymformulierungen den Vorteil, dass der Wassergehalt in wasserarmen Reinigungsmittelformulierungen weiter verringert werden kann, da flüssige Enzymformulierungen üblicherweise einen hohen Wassergehalt aufweisen.

In einem ersten Aspekt richtet sich die vorliegende Erfindung daher auf ein flüssiges Reinigungsmittel, insbesondere maschinelles Geschirrspülmittel gemäß Anspruch 1, enthaltend
(1) mindestens eine flüssige Enzymformulierung, die mindestens eine Amylase (A1) und optional mindestens eine Protease (P1) enthält; und
(2) mindestens eine feste Enzymformulierung, vorzugsweise in Form eines Granulats, die mindestens eine Protease (P2) und/oder mindestens eine Amylase (A2) enthält, wobei die feste Enzymformulierung in dem flüssigen Geschirrspülmittel homogen suspendiert ist,
wobei die Amylase (A1) ausgewählt ist aus
a) einer AA560 α-Amylase aus Bacillus sp. oder ein funktionales Fragment davon; und/oder
b) einer Amylase, die eine Aminosäuresequenz umfasst, die zu der in SEQ ID NO:2 angegebenen Aminosäuresequenz über deren Gesamtlänge zu mindestens 80% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine der Deletionen D183* and G184* in der Zählung gemäß SEQ ID NO:2 aufweist; und/oder
c) einer Amylase, die eine Aminosäuresequenz umfasst, die zu der in SEQ ID NO:2 angegebenen Aminosäuresequenz über deren Gesamtlänge zu mindestens 80% identisch ist und in der Zählung gemäß SEQ ID NO:2 folgende Aminosäuresubstitutionen und/oder Deletionen aufweist:
   (i) M9L + M323T;
   (ii) M9L + M202L/T/V/I + M323T;
   (iii) M9L + N195F + M202L/T/V/I + M323T;
   (iv) M9L + R118K + D183* + G184* + R320K + M323T + R458K;
   (v) M9L + R118K + D183* + G184* + M202L/T/V/I + R320K + M323T + R458K;
   (vi) M9L + G149A + G182T + G186A + M202L + T257I + Y295F + N299Y + M323T + A339S + E345R;
   (vii) M9L + G149A + G182T + G186A + M202I + T257I + Y295F + N299Y + M323T + A339S + E345R;
   (viii) M9L + R118K + G149A + G182T + D183* + G184* + G186A + M202L + T257I + Y295F + N299Y + R320K + M323T + A339S + E345R + R458K;
   (ix) M9L + R118K + G149A + G182T + D183* + G184* + G186A + N195F + M202L + T257I + Y295F + N299Y + R320K + M323T + A339S + E345R + R458K;
   (x) M9L + R118K + G149A + G182T + D183* + G184* + G186A + M202I + T257I + Y295F + N299Y + R320K + M323T + A339S + E345R + R458K;
   (xi) M9L + R118K + D183* + D184* + N195F + M202L + R320K + M323T + R458K;
   (xii) M9L + R118K + D183* + D184* + N195F + M202T + R320K + M323T + R458K;
   (xiii) M9L + R118K + D183* + D184* + N195F + M202I + R320K + M323T + R458K;
   (xiv) M9L + R118K + D183* + D184* + N195F + M202V + R320K + M323T + R458K;
   (xv) M9L + R118K + N150H + D183* + D184* + N195F + M202L + V214T + R320K + M323T + R458K; oder
   (xvi) M9L + R118K + D183* + D184* + N195F + M202L + V214T + R320K + M323T + E345N + R458K.

Ein weiterer Gegenstand der vorliegenden Erfindung ist die Verwendung eines hierin beschriebenen Reinigungsmittels als Geschirrspülmittels, vorzugsweise als maschinelles Geschirrspülmittel.

Noch ein weiterer Aspekt betrifft die ein Verfahren zur Reinigung von Geschirr in einer automatischen Geschirrspülmaschine, wobei ein Reinigungsmittel wie hierin beschrieben eingesetzt und vorzugsweise während des Durchlaufens eines Geschirrspülprogramms vor Beginn des Hauptspülgangs oder im Verlaufe des Hauptspülgangs in den Innenraum der Geschirrspülmaschine eindosiert wird.

In verschiedenen Ausführungsformen der Erfindung werden bei dem Geschirrspülverfahren Temperaturen eingesetzt, die niedriger sind als die üblicherweise verwendeten Temperaturen.

"Homogen suspendiert", wie hierin verwendet, bezieht sich auf die eine Suspension, die die feste Enzymzubereitung in Form von stabil und gleichmäßig dispergierten Partikeln enthält. "Stabil dispergiert" bedeutet, dass sich die Partikel bei Lagerung bei Raumtemperatur über einen Zeitraum von mindestens einer Woche nicht sedimentieren oder aufrahmen.

"Niedrige Temperaturen" oder "Temperaturen, die niedriger sind als die üblicherweise verwendeten Temperaturen", wie hierin im Zusammenhang mit Geschirrspülverfahren verwendet, bezieht sich vorzugsweise auf Temperaturen unter 60°C, insbesondere unter 55°C, noch bevorzugter 50°C oder niedriger, besonders bevorzugt 45°C oder niedriger und am bevorzugtesten 40°C oder niedriger. Diese Temperaturangaben beziehen sich auf die in den Reinigungsschritten eingesetzten Zieltemperaturen.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist. Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

In verschiedenen Ausführungsformen der Erfindung umfasst das Reinigungsmittel mindestens eine erste (A2) und/oder mindestens eine zweite Amylase (A1), wobei
(A) die erste Amylase (A2) in der festen Enzymformulierung enthalten ist; und
(B) die zweite Amylase (A1) in der flüssigen Enzymformulierung enthalten ist.

Die eingesetzten Amylasen sind insbesondere alkalische a-Amylasen. Sie wirken als Hydrolasen und spalten die α-(1-4)-Glykosidbindung von Polysacchariden, insbesondere Stärken wie Amylose, und bewirken dadurch den Abbau stärkehaltiger Anschmutzungen auf dem Reinigungsgut. Als Spaltprodukte entstehen dabei Dextrine und daraus Maltose, Glucose und verzweigte Oligosaccharide. Ihr pH-Optimum liegt meist im deutlich alkalischen Bereich.

In verschiedenen Ausführungsformen der Erfindung ist die die erste Amylase eine α-Amylase aus *Bacillus sp.* No. 707 oder ein funktionales Fragment oder eine Variante davon. In verschiedenen weiteren Ausführungsformen ist die zweite Amylase eine AA560 α-Amylase aus *Bacillus sp.* oder ein funktionales Fragment oder eine Variante davon.

Die Wildtyp-Sequenzen der reifen (maturen) α-Amylase aus *Bacillus sp.* No. 707 bzw. der reifen (maturen) AA560 α-Amylase aus *Bacillus sp.* sind in SEQ ID NO:1 bzw. SEQ ID NO:2 angegeben.

"Verschieden", wie hierin mit Bezug auf die Enzyme verwendet, bezieht sich auf Enzyme, die sich in ihrer Aminosäuresequenz unterscheiden. In verschiedenen Ausführungsformen stammen voneinander verschiedene Enzyme aus unterschiedlichen Arten von Organismen oder unterscheiden sich voneinander durch, beispielsweise künstlich erzeugte, Mutationen.

"Variante", wie hierin in Zusammenhang mit Enzymen verwendet, bezieht sich auf natürliche oder artifiziell erzeugte Variationen eines nativen Enzyms, die eine gegenüber der Referenzform abgewandelte Aminosäuresequenz aufweisen. Eine solche Variante kann einzelne oder mehrere Punktmutationen, d.h. Substitutionen einer natürlicherweise an der entsprechenden Position vorkommenden Aminosäure durch eine andere, Insertionen (Einfügen von einer oder mehreren Aminosäuren) und/oder Deletionen (Entfernen von einer oder mehreren Aminosäuren) aufweisen, insbesondere eine oder mehrere Punktmutationen. Derartige Varianten haben vorzugsweise mindestens 50%, vorzugsweise 60% oder mehr, noch bevorzugter 70%, 80%, 90%, 100% oder mehr der Enzymaktivität der Referenzform. In verschiedenen Ausführungsformen hat eine derartige Variante eine Aminosäuresequenz, die zu der als Referenz dienenden Sequenz über deren Gesamtlänge zu mindestens 70%, vorzugsweise 75%, 80%, 85%, 90%, 95%, 96%, 97%, 98% oder 99% identisch ist. Die Varianten haben vorzugsweise dieselbe Länge wie die Referenzsequenz. Varianten können sich gegenüber der Referenzform durch verbesserte Eigenschaften auszeichnen, wie beispielsweise höhere Enzymaktivität, höhere Stabilität, geänderte Substratspezifität, etc. Eingesetzt werden nur solche Varianten, die enzymatische Aktivität aufweisen. "Enzymatische Aktivität", wie in diesem Zusammenhang verwendet, bedeutet insbesondere, dass die entsprechenden Enzyme mindestens 50%, vorzugsweise mindestens 90% der katalytischen Aktivität ihres Referenzenzymes aufweisen.

"Fragment", wie hierin in Zusammenhang mit Enzymen verwendet, bezieht sich auf gegenüber dem Referenz-Enzym N-terminal und/oder C-terminal um jeweils eine oder mehrere Aminosäuren verkürzte Polypeptide. Eingesetzt werden nur solche Fragmente, die enzymatische Aktivität aufweisen. "Enzymatische Aktivität", wie in diesem Zusammenhang verwendet, bedeutet insbesondere, dass die entsprechenden Enzyme mindestens 50%, vorzugsweise mindestens 90% der katalytischen Aktivität ihres Referenzenzymes aufweisen.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essenzielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Als erste Amylase im Sinne der vorliegenden Erfindung wird in verschiedenen bevorzugten Ausführungsformen eine Amylase eingesetzt, die eine Aminosäuresequenz umfasst, die zu der in SEQ ID NO:1 angegebenen Aminosäuresequenz über deren Gesamtlänge zu mindestens 80% identisch ist und optional mindestens eine Aminosäuresubstitution an einer der Positionen 172, 202, 208, 255 und 261 in der Zählung gemäß SEQ ID NO:1 aufweist, insbesondere ausgewählt aus der Gruppe bestehend aus M202L, M202V, M202S, M202T, M202I, M202Q, M202W, S255N und R172Q.

In bevorzugten Ausführungsformen der Erfindung wird somit als erste Amylase eine Variante von der α-Amylase aus *Bacillus sp.* No. 707 mit der in SEQ ID NO:1 angegebenen Aminosäuresequenz eingesetzt, die zu der in SEQ ID NO:1 angegebenen Aminosäuresequenz über deren Gesamtlänge zu mindestens 80% identisch ist und mindestens eine Aminosäuresubstitution an einer der Positionen 172, 202, 208, 255 und 261 in der Zählung gemäß SEQ ID NO:1 aufweist. Bevorzugt werden Amylasen eingesetzt, die an zwei, bevorzugt drei, der vorstehend genannten Positionen eine Aminosäuresubstitution aufweisen, insbesondere eine Substitution an Position 202 ausgewählt aus M202L, M202V, M202S, M202T, M202I, M202Q, M202W, eine Substitution an Position 255, insbesondere S255N, und eine Substitution an Position 172, insbesondere R172Q. Ganz besonders bevorzugt sind die M202L und M202T Mutanten.

Weitere einsetzbare Varianten sind solche, die eine Aminosäuresequenz besitzen, die zu der in SEQ ID NO:1 angegebenen Aminosäuresequenz mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% oder 99% identisch ist, wobei die Positionen 172, 202 und 255 vorzugsweise wie oben beschrieben substituiert sind. Derartige Varianten können beispielsweise eine Verkürzung des C-Terminus einschließen, beispielsweise um 1-20 Aminosäure, oder eine Deletion von einer oder mehreren Aminosäuren, insbesondere an den Positionen 181, 182, 183 und 184 in der Zählung gemäß SEQ ID NO:1, wobei allerdings die enzymatische Aktivität beibehalten wird, d.h. die Aktivität der Variante mindestens 60% der Aktivität des Enzyms mit der Aminosäuresequenz von SEQ ID NO:1 beträgt. Geeignete Amylasen werden auch in der WO 2008/112459 A2 beschrieben, deren gesamte Offenbarung hierin durch Bezugnahme eingeschlossen ist.

Die zweite Amylase ist von der ersten Amylase verschieden, d.h. eine Amylase, die sowohl unter die Definition für die erste Amylase als auch für die der zweiten Amylase fällt, kann nicht als erste und als zweite Amylase gleichzeitig gezählt werden.

In verschiedenen Ausführungsformen der Erfindung umfasst die zweite Amylase eine Aminosäuresequenz, die zu der in SEQ ID NO:2 angegebenen Aminosäuresequenz über deren Gesamtlänge zu mindestens 80% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine der Deletionen D183* and G184* in der Zählung gemäß SEQ ID NO:2 aufweist

In besonders bevorzugten Ausführungsformen weist zweite Amylase in der Zählung gemäß SEQ ID NO:2 folgende Aminosäuresubstitutionen und/oder Deletionen auf:
(i) M9L + M323T;
(ii) M9L + M202L/T/V/I + M323T;
(iii) M9L + N195F + M202L/T/V/I + M323T;
(iv) M9L + R118K + D183* + G184* + R320K + M323T + R458K;
(v) M9L + R118K + D183* + G184* + M202L/T/V/I + R320K + M323T + R458K;
(vi) M9L + G149A + G182T + G186A + M202L + T257I + Y295F + N299Y + M323T + A339S + E345R;
(vii) M9L + G149A + G182T + G186A + M202I + T257I + Y295F + N299Y + M323T + A339S + E345R;
(viii) M9L + R118K + G149A + G182T + D183* + G184* + G186A + M202L + T257I + Y295F + N299Y + R320K + M323T + A339S + E345R + R458K;
(ix) M9L + R118K + G149A + G182T + D183* + G184* + G186A + N195F + M202L + T257I + Y295F + N299Y + R320K + M323T + A339S + E345R + R458K;
(x) M9L + R118K + G149A + G182T + D183* + G184* + G186A + M202I + T257I + Y295F + N299Y + R320K + M323T + A339S + E345R + R458K;
(xi) M9L + R118K + D183* + D184* + N195F + M202L + R320K + M323T + R458K;
(xii) M9L + R118K + D183* + D184* + N195F + M202T + R320K + M323T + R458K;
(xiii) M9L + R118K + D183* + D184* + N195F + M202I + R320K + M323T + R458K;
(xiv) M9L + R118K + D183* + D184* + N195F + M202V + R320K + M323T + R458K;
(xv) M9L + R118K + N150H + D183* + D184* + N195F + M202L + V214T + R320K + M323T + R458K; oder
(xvi) M9L + R118K + D183* + D184* + N195F + M202L + V214T + R320K + M323T + E345N + R458K.

Eine besonders bevorzugte zweite Amylase ist die Variante, die unter dem Handelnamen Stainzyme Plus^{™} kommerziell erhältlich ist (Novozymes A/S, Bagsvaerd, Dänemark).

Bevorzugt werden im Rahmen der vorliegenden Erfindung Kombinationen einer ersten Amylase, die zu der in SEQ ID NO:1 angegebenen Aminosäuresequenz über deren Gesamtlänge zu mindestens 80% identisch ist und mindestens eine Aminosäuresubstitution an einer der Positionen 172, 202, 208, 255 und 261 in der Zählung gemäß SEQ ID NO: 1 aufweist, insbesondere eine Substitution an Position 202 ausgewählt aus M202L, M202V, M202S, M202T, M202I, M202Q, M202W, eine Substitution an Position 255, insbesondere S255N, und eine Substitution an Position 172, insbesondere R172Q, und einer zweiten Amylase, die zu der in SEQ ID NO:2 angegebenen Aminosäuresequenz über deren Gesamtlänge zu mindestens 80% identisch ist und folgende Aminosäuresubstitutionen und/oder Deletionen aufweist
(i) M9L + M323T;
(ii) M9L + M202L/T/V/I + M323T;
(iii) M9L + N195F + M202L/T/V/I + M323T;
(iv) M9L + R118K + D183* + G184* + R320K + M323T + R458K;
(v) M9L + R118K + D183* + G184* + M202L/T/V/I + R320K + M323T + R458K;
(vi) M9L + G149A + G182T + G186A + M202L + T257I + Y295F + N299Y + M323T + A339S + E345R;
(vii) M9L + G149A + G182T + G186A + M202I + T257I + Y295F + N299Y + M323T + A339S + E345R;
(viii) M9L + R118K + G149A + G182T + D183* + G184* + G186A + M202L + T257I + Y295F + N299Y + R320K + M323T + A339S + E345R + R458K;
(ix) M9L + R118K + G149A + G182T + D183* + G184* + G186A + N195F + M202L + T257I + Y295F + N299Y + R320K + M323T + A339S + E345R + R458K;
(x) M9L + R118K + G149A + G182T + D183* + G184* + G186A + M202I + T257I + Y295F + N299Y + R320K + M323T + A339S + E345R + R458K;
(xi) M9L + R118K + D183* + D184* + N195F + M202L + R320K + M323T + R458K;
(xii) M9L + R118K + D183* + D184* + N195F + M202T + R320K + M323T + R458K;
(xiii) M9L + R118K + D183* + D184* + N195F + M202I + R320K + M323T + R458K;
(xiv) M9L + R118K + D183* + D184* + N195F + M202V + R320K + M323T + R458K;
(xv) M9L + R118K + N150H + D183* + D184* + N195F + M202L + V214T + R320K + M323T + R458K; oder
(xvi) M9L + R118K + D183* + D184* + N195F + M202L + V214T + R320K + M323T + E345N + R458K.

In verschiedenen Ausführungsformen werden diese Kombinationen von Amylasen im Massenverhältnis bezogen auf Aktivprotein von 10:1 bis 1:10, vorzugsweise 5:1 bis 1:5, insbesondere von 2:1 bis 1:2, beispielsweise 2:3 bis 3:2, besonders bevorzugt zu gleichen Teilen eingesetzt.

Die hierin beschriebenen Reinigungsmittel können in bevorzugten Ausführungsformen mindestens eine erste (P2) und mindestens eine zweite Protease (P1)enthält, wobei
(A) die erste Protease (P2) in der festen Enzymformulierung enthalten ist; und
(B) die zweite Protease (P1) in der flüssigen Enzymformulierung enthalten ist.

Die Reinigungsmittel können diese Proteasen entweder allein oder, vorzugsweise, in Kombination mit den oben beschriebenen Amylasen enthalten. Dabei können die verschiedenen Enzyme jeweils separat in einer flüssigen oder festen Rezeptur oder jeweils gemeinsam in einer festen bzw. einer flüssigen Rezeptur (d.h. die erste Amylase und erste Protease in einer gemeinsamen festen Formulierung und/oder die zweite Amylase und zweite Protease in einer gemeinsamen flüssigen Formulierung) formuliert sein.

Es kann bevorzugt sein, die erste Protease in der festen Enzymformulierung und die zweite Amylase in der flüssigen Enzymformulierung zu formulieren.

Es kann ebenfalls bevorzugt sein, die erste Amylase in der festen Enzymformulierung und die zweite Protease in der flüssigen Enzymformulierung zu formulieren.

Bevorzugte Kombination enthalten sowohl in der flüssigen Phase als auch in der festen Phase jeweils eine entsprechende Amylase vorhanden ist und in einer der beiden Phasen dann eine entweder in der flüssigen oder der festen Phase eine entsprechende Protease.

Bevorzugt kann es ebenfalls sein, dass sowohl die flüssige als auch die feste Phase jeweils eine der entsprechenden Amylase und eine entsprechende Protease enthält.

Die Proteasen sind insbesondere alkalische Serin-Proteasen. Sie wirken als unspezifische Endopeptidasen, das heißt, sie hydrolysieren beliebige Säureamidbindungen, die im Inneren von Peptiden oder Proteinen liegen und bewirken dadurch den Abbau proteinhaltiger Anschmutzungen auf dem Reinigungsgut. Ihr pH-Optimum liegt meist im deutlich alkalischen Bereich.

In verschiedenen Ausführungsformen der Erfindung umfasst die mindestens eine erste Protease eine Protease aus *Bacillus alkalophilus* PB92 oder ein funktionales Fragment oder eine Variante davon. Die Sequenz der reifen (maturen) Protease aus *Bacillus alkalophilus* PB92 (Wildtyp) ist in SEQ ID NO:4 angegeben. In bevorzugten Ausführungsformen kann diese erste Protease eine Aminosäuresequenz aufweisen, die zu der in SEQ ID NO:4 angegebenen Aminosäuresequenz über deren Gesamtlänge zu mindestens 80%, bevorzugt zu mindestens 90%, insbesondere zu 100% identisch ist und die optional mindestens eine Aminosäuresubstitution an einer der folgenden Positionen 32, 33, 48-54, 58-62, 94-107, 116, 123-133, 150, 152-156, 158-161, 164, 169, 175-186, 197, 198, 203-216 in der Zählung gemäß SEQ ID NO:4 aufweist.

Weitere einsetzbare Varianten sind solche, die eine Aminosäuresequenz besitzen, die zu der in SEQ ID NO:4 angegebenen Aminosäuresequenz zu mindestens 80% und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist und in der Zählung gemäß SEQ ID NO:4 an mindestens eine der folgenden Positionen 32, 33, 48-54, 58-62, 94-107, 116, 123-133, 150, 152-156, 158-161, 164, 169, 175-186, 197, 198, 203-216 eine Aminosäuresubstitution trägt.

Es kann bevorzugt eine Variante der Protease mit der in SEQ ID NO:4 angegebenen Aminosäuresequenz eingesetzt werden, die zu der in SEQ ID NO:4 angegebenen Aminosäuresequenz über deren Gesamtlänge zu mindestens 80% identisch ist und die mindestens eine Aminosäuresubstitution an einer der Positionen 116, 126, 127, 128 und 160 in der Zählung gemäß SEQ ID NO:4 aufweist. Bevorzugt werden Proteasen eingesetzt, die an zwei, bevorzugt drei oder mehr, insbesondere vier der vorstehend genannten Positionen eine Aminosäuresubstitution aufweisen.

Weitere einsetzbare Varianten sind solche, die eine Aminosäuresequenz besitzen, die zu der in SEQ ID NO:4 angegebenen Aminosäuresequenz mindestens zu 81%, 82%, 83%, 84%, 85%, 86%, 87%, 88%, 89%, 90%, 91%, 92%, 93%, 94%, 95%, 96%, 97%, 98% oder 99% identisch ist, und die mindestens eine Aminosäuresubstitution an einer der folgenden Positionen 116, 126, 127, 128 und 160 aufweisen.

Besonders bevorzugt weist eine derartige Protease eine Aminosäuresequenz auf, die zu der in SEQ ID NO:4 angegebenen Aminosäuresequenz über deren Gesamtlänge zu mindestens 80% identisch ist und die in der Zählung gemäß SEQ ID NO:4 eine der folgenden Kombinationen von Aminosäuresubstitutionen aufweist:
(i) G116V + S126L + P127Q + S128A
(ii) G116V + S126N + P127S + S128A + S160D
(iii) G116V + S126L + P127Q + S128A + S160D
(iv) G116V + S126V + P127E + S128K
(v) G116V + S126V + P127M + A160D
(vi) S128T
(vii) G116V + S126F + P127L + S128T
(viii) G116V + S126L + P127N + S128V
(ix) G116V + S126F + P127Q
(x) G116V + S126V + P127E + S128K +S160D
(xi) G116V + S126R + P127S + S128P
(xii) S126R + P127Q + S128D
(xiii) S126C + P127R + S128D; oder
(xiv) S126C + P127R + S128G.

Bevorzugt weist eine derartige Protease eine Aminosäuresequenz auf, die in der Zählung gemäß SEQ ID NO:4 mindestens eine, bevorzugt mehrere, insbesondere jede der folgenden Aminosäuresubstitutionen G116V, S126L, P127Q und/oder S128A aufweist und an allen anderen Stellen zu der in SEQ ID NO:4 angegebenen Aminosäuresequenz über deren Gesamtlänge zu mindestens 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% insbesondere zu 100 % identisch ist. Besonders bevorzugt ist eine Protease, die eine Aminosäuresequenz aufweist, die ausgehend von der Aminosäuresequenz mit der SEQ ID NO:4 durch die Aminosäuresubstitutionen G116V, S126L, P127Q und S128A gemäß der Zählung nach SEQ ID NO:4 erhältlich ist. Eine derartige Protease kann die in SEQ ID NO:8 angegebene Aminosäuresequenz aufweisen.

Die mindestens eine zweite Protease wird vorzugsweise ausgewählt aus der Gruppe bestehend aus einem Subtilisin 309 aus *Bacillus lentus* oder einem funktionalen Fragment oder einer Variante davon und einer alkalischen Protease aus *Bacillus lentus* DSM 5483 oder einem funktionalen Fragment oder einer Variante davon. Es können ebenfalls Kombinationen von mehreren der vorgenannten Enzyme eingesetzt werden.

Die Sequenzen der reifen (maturen) Protease Subtilisin 309 aus *Bacillus lentus* bzw. der reifen (maturen) alkalischen Protease aus *Bacillus lentus* DSM 5483 sind in SEQ ID NO:3 bzw. SEQ ID NO:5 angegeben.

In verschiedenen Ausführungsformen der Erfindung umfasst die zweite Protease ein Subtilisin 309 aus *Bacillus lentus* oder ein funktionales Fragment oder eine Variante davon, umfassend eine Aminosäuresequenz, die zu der in SEQ ID NO:3 angegebenen Aminosäuresequenz über deren Gesamtlänge zu mindestens 80%, bevorzugt zu mindestens 90%, insbesondere zu 100% identisch ist und mindestens eine Aminosäuresubstitution an einer der Positionen 9, 15, 66, 212 und 239 in der Zählung gemäß SEQ ID NO:3 aufweist. Bevorzugt sind solche, die an zwei, bevorzugt drei, insbesondere vier, ganz besonders bevorzugt fünf der vorstehend genannten Positionen eine Aminosäuresubstitution aufweisen.

Weitere einsetzbare Varianten sind solche, die eine Aminosäuresequenz besitzen, die zu der in SEQ ID NO:3 angegebenen Aminosäuresequenz mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% oder 99% identisch ist, wobei eine oder mehrere der Positionen 9, 15, 66, 212 und 239 substituiert sind, d.h. die Aminosäure an diesen Positionen nicht der entsprechenden Aminosäure in SEQ ID NO:3 entspricht.

Besonders bevorzugt verwendet wird eine Variante mit mindestens einer, bevorzugt zwei, insbesondere drei, besonders bevorzugt vier oder ganz besonders bevorzugt 5 der Aminosäuresubstitutionen ausgewählt aus S9R, A15T, V66A, N212D und Q239R bezogen auf die Zählung nach SEQ ID NO:3. Bevorzugt sind die folgenden Kombinationen: S9R+V66A+N212D+Q239R, S9R+A15T+N212D+Q239R, S9R+A15T+V66A+Q239R, S9R+A15T+V66A+N212D, A15T+V66A+N212D+Q239R; S9R+A15T+V66A, S9R+A15T+N212D, S9R+A15T+Q239R, S9R+N212D+Q239R, S9R+V66A+N212D, S9R+V66A+Q239R, A15T+V66A+N212D, A15T+V66A+Q239R, A15T+N212D+Q239R, V66A+N212D+Q239R; S9R+A15T, S9R+V66A, S9R+N212D, S9R+Q239R, A15T+V66A, A15T+N212D, A15T+Q239R, V66A+N212D, V66A+Q239R, N212D+Q239R. Eine Variante, die alle vorstehend genannten Änderungen umfasst, hat die in SEQ ID NO:6 angegebene Aminosäuresequenz (S9R+A15T+V66A+N212D+Q239R).

Ebenfalls besonders bevorzugt werden Varianten der Protease mit der in SEQ ID NO:3 angegebenen Aminosäuresequenz, die eine Aminosäuresubstitution an der Position 99 und eine Insertion von einer Aminosäure zwischen den Aminosäuren an Positionen 99 und 100 in der Zählung gemäß SEQ ID NO:3 aufweisen, vorzugsweise ausgewählt aus S99A und/oder S99_G100InsD. Weitere einsetzbare Varianten sind solche, die eine Aminosäuresequenz besitzen, die zu der in SEQ ID NO:3 angegebenen Aminosäuresequenz mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% oder 99% identisch ist, wobei diese Varianten eine oder beide der oben genannten Mutationen an den Positionen 99 und 100 aufweisen. Bevorzugt sind solche, die beide Mutationen aufweisen. Eine derartige Variante, hat die in SEQ ID NO:7 angegebene Aminosäuresequenz.

In verschiedenen noch weiteren Ausführungsformen der Erfindung umfasst die zweite Protease eine alkalische Protease aus *Bacillus lentus* DSM 5483 oder ein funktionales Fragment oder eine Variante davon, die eine Aminosäuresequenz aufweist, die zu der in SEQ ID NO:5 angegebenen Aminosäuresequenz über deren Gesamtlänge zu mindestens 80%, bevorzugt zu mindestens 90%, insbesondere zu 100% identisch ist und die optional mindestens eine Aminosäuresubstitution an einer, zwei, drei oder vier der folgenden Positionen 3, 4, 99 und 199 in der Zählung gemäß SEQ ID NO:5 aufweist. Bevorzugt werden Proteasen eingesetzt, die an zwei, bevorzugt drei oder mehr, insbesondere vier der vorstehend genannten Positionen eine Aminosäuresubstitution aufweisen.

Weitere einsetzbare Varianten sind solche, die eine Aminosäuresequenz besitzen, die zu der in SEQ ID NO:5 angegebenen Aminosäuresequenz mindestens zu 81%, 82%, 83%, 84%, 85%, 86%, 87%, 88%, 89%, 90%, 91%, 92%, 93%, 94%, 95%, 96%, 97%, 98% oder 99% identisch ist, und die mindestens eine Aminosäuresubstitution an einer der folgenden Positionen 3, 4, 99 und 199 aufweisen.

Besonders bevorzugt weist eine derartige Protease eine Aminosäuresequenz auf, die zu der in SEQ ID NO:5 angegebenen Aminosäuresequenz über deren Gesamtlänge zu mindestens 80% identisch ist und die in der Zählung gemäß SEQ ID NO:5 die Aminosäuresubstitution R99E oder R99D, sowie optional zusätzlich mindestens eine oder zwei, vorzugsweise alle drei der Aminosäuresubstitutionen S3T, V4I und V199I.

Bevorzugt weist eine derartige Protease eine Aminosäuresequenz auf, die in der Zählung gemäß SEQ ID NO:5 mindestens eine, bevorzugt mehrere, insbesondere jede der folgenden Aminosäuresubstitution R99E/R99D, S3T, V4I und/oder V199I aufweist und an allen anderen Stellen zu der in SEQ ID NO:5 angegebenen Aminosäuresequenz über deren Gesamtlänge zu mindestens 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% insbesondere zu 100 % identisch ist. Besonders bevorzugt ist eine Protease, die eine Aminosäuresequenz aufweist, die ausgehend von der Aminosäuresequenz mit der SEQ ID NO:5 durch eine oder mehrere der Aminosäuresubstitutionen R99E/R99D, S3T, V4I und V199I gemäß der Zählung nach SEQ ID NO:5 erhältlich ist. Eine derartige Protease kann die in einer der SEQ ID Nos:9-10 angegebene Aminosäuresequenz aufweisen.

Es ist erfindungsgemäß besonders bevorzugt, eine Kombination einer ersten und einer zweiten Protease einzusetzen. Besonders bevorzugt ist dabei eine erste Protease, die eine Aminosäuresequenz aufweist, die ausgehend von der Aminosäuresequenz mit der SEQ ID NO:4 durch die Aminosäuresubstitutionen G116V, S126L, P127Q und S128A gemäß der Zählung nach SEQ ID NO:4 erhältlich ist. Eine derartige Protease kann die in SEQ ID NO:8 angegebene Aminosäuresequenz aufweisen. Als zweite Protease wird dabei insbesondere eine Variante der Protease mit der Aminosäuresequenz gemäß SEQ ID NO:9 eingesetzt.

In verschiedenen Ausführungsformen werden diese Kombinationen von zwei Proteasen im Massenverhältnis bezogen auf Aktivprotein von 10:1 bis 1:10, vorzugsweise 5:1 bis 1:5, insbesondere von 2:1 bis 1:2, beispielsweise 2:3 bis 3:2, besonders bevorzugt zu gleichen Teilen eingesetzt.

Die Reinigungsmittel der Erfindung können die feste Enzymformulierung in einer Menge von 0,01 bis 5 Gew.-%, vorzugsweise 0,2 bis 2 Gew.-%, bezogen auf das Gesamtgewicht des Reinigungsmittels enthalten. Dabei kann die feste Enzymformulierung insbesondere einen Aktivenzymgehalt von 2 bis 20 Gew.-% aufweisen.

Die flüssige Enzymformulierung kann in den Reinigungsmitteln in einer Menge von 0,01 bis 8 Gew.-%, vorzugsweise 0,3 bis 6 Gew.-%, bezogen auf das Gesamtgewicht des Reinigungsmittels enthalten sein. Dabei kann die flüssige Enzymformulierung insbesondere einen Aktivenzymgehalt von 1 bis 6 Gew.-% aufweisen.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden. Die Bestimmung der Aktivproteinkonzentration erfolgt diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Generell kann das Reinigungsmittel die flüssige Enzymformulierung und die feste Enzymformulierung in einem Massenverhältnis von 10:1 bis 1:10, vorzugsweise 1:1, enthalten.

Überraschenderweise zeigen die hierin beschriebenen Kombinationen von flüssig und fest formulierten Amylasen bzw. Proteasen die Eigenschaft, die Leistung des Reinigungsmittels, bevorzugt des Geschirrspülmittels zu verbessern, indem sie zu einer verbesserten Reinigungsleistung an enzymsensitiven Anschmutzungen führen.

Dabei ist in der Regel unter der Verbesserung der Reinigungsleistung zu verstehen, dass bei Verwendung der hierin beschriebenen Reinigungsmittel, insbesondere der Geschirrspülmittel die Entfernung von Anschmutzungen auf harten Oberflächen, insbesondere Geschirr, bei dessen Reinigung bevorzugt in einer automatischen Geschirrspülmaschine im Vergleich zu der Verwendung von Reinigungsmitteln, bevorzugt Geschirrspülmitteln, die die hierin beschriebenen Enzymkombinationen nicht enthalten, merklich verbessert ist.

Die einzusetzenden Enzyme sind in den jeweiligen Formulierungen ferner zusammen mit Begleitstoffen, etwa aus der Fermentation, oder mit Stabilisatoren konfektioniert.

Die Enzyme können besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung ist eine Inhibierung der Proteolyse besonders bevorzugt. Die beschriebenen Mittel können zu diesem Zweck Stabilisatoren enthalten. Reinigungsaktive Enzyme werden typischerweise schon in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Wie aus den vorherigen Ausführungen ersichtlich, bildet das Enzym-Protein nur einen Bruchteil des Gesamtgewichts üblicher Enzym-Zubereitungen. Typischerweise eingesetzte Enzym-Zubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 15 Gew.-% des Enzymproteins.

In verschiedenen Ausführungsformen der Erfindung ist die feste Enzymzubereitung ein Granulat. Die Granulatpartikel können farbig sein oder lichtreflektierende Eigenschaften aufweisen. Die Partikel haben üblicherweise eine mittlere Partikelgröße (Siebanalyse) im Bereich von 1 µm bis 700 µm.

Bevorzugte flüssige Mittel weisen Dichten von 0,5 bis 2,0 g/cm³, insbesondere 0,7 bis 1,7 g/cm³, insbesondere 1,0 bis 1,5 g/cm³ auf. Die Dichtedifferenz zwischen den Granulaten und der flüssigen Phase des Mittels beträgt vorzugsweise nicht mehr als 10% der Dichte einer der beiden und ist insbesondere so gering, dass die erfindungsgemäßen Granulate und vorzugsweise auch gegebenenfalls sonstige in den Mitteln enthaltene Feststoffpartikel in der Flüssigphase schweben.

In verschiedenen Ausführungsformen ist die flüssige Enzymzubereitung eine Lösung, insbesondere eine wässrige Lösung.

Die hierin beschriebenen Reinigungsmittel sind (homogene) Lösungen, in denen die feste Enzymformulierungen stabil suspendiert und die flüssigen Enzymzubereitungen vorzugsweise gelöst sind.

In einer bevorzugten Ausführungsform der Erfindung liegt das Reinigungsmittel, insbesondere das maschinelle Geschirrspülmittel, in einer vorportionierten Form vor. In einer Ausführungsform der Erfindung weist das Reinigungsmittel mehrere räumlich voneinander getrennte Zusammensetzungen auf, wodurch es möglich ist, nicht kompatible Inhaltsstoffe voneinander zu trennen, oder Zusammensetzungen in Kombination anzubieten, welche zu unterschiedlichen Zeitpunkten in der Geschirrspülmaschine zum Einsatz kommen. Dies ist besonders vorteilhaft, wenn die maschinellen Geschirrspülmittel in vorportionierter Form vorliegen.

Die Reinigungsmittel der Erfindung sind vorzugsweise flüssige, wasserarme Zusammensetzungen. Dabei handelt es sich bei den erfindungsgemäßen Reinigungsmitteln vorzugsweise um Geschirrspülmittel, insbesondere um maschinelle Geschirrspülmittel.

Der Ausdruck "wasserarm", wie hierin verwendet, bedeutet, dass die derart charakterisierte Zusammensetzung weniger als 25 Gew.-% Wasser, vorzugsweise weniger als 20 Gew.-% Wasser enthält. Insbesondere fallen unter diesen Begriff Zusammensetzungen, die 1 bis 20 Gew.-% Wasser, 1 bis 15 Gew.-% Wasser, 5-15 Gew.-% Wasser oder 10 bis weniger als 20 Gew.-% Wasser enthalten.

Der Wassergehalt wie hierin definiert bezieht sich auf den mittels der Karl Fischer Titration ermittelten Wassergehalt.

"Flüssig", wie hierin in Bezug auf das erfindungsgemäße Reinigungsmittel verwendet, schließt alle fließfähigen Zusammensetzungen ein und erfasst insbesondere auch Gele und pastöse Zusammensetzungen.

"Mindestens ein", wie hierin verwendet, bedeutet 1 oder mehr, beispielsweise 1, 2, 3, 4, 5, oder mehr.

In verschiedenen Ausführungsformen enthält das Reinigungsmittel mindestens einen mehrwertigen Alkohol, insbesondere ausgewählt aus Glycerin und Mischungen von Glycerin und 1,2-Propylenglykol enthält. Die mehrwertigen Alkohole ermöglichen auch bei geringer Wassermenge, insbesondere bei einer Begrenzung der Wassermenge auf 20 Gew.-%, die Einarbeitung anderer Bestandteile in die Reinigungsmittelformulierung.

Die Gesamtmenge an in erfindungsgemäßen Reinigungsmitteln eingesetzten mehrwertigem Alkohol oder Alkoholgemischen liegt vorzugsweise bei mindestens 20 Gew.-%, insbesondere bei mindestens 25 Gew.-%, besonders bevorzugt bei mindestens 28 Gew.-%, vor allem bei mindestens 30 Gew.-%. Bevorzugte Mengenbereiche sind hierbei 20 bis 50 Gew.-%, insbesondere 25 bis 45 Gew.-%, vor allem 28 bis 40 Gew.-%.

Der mehrwertige Alkohol ist vorzugsweise ausgewählt aus Glycerin, Sorbitol und Mischungen von Glycerin und/oder Sorbitol mit 1,2-Propylenglykol. Glycerin wird in erfindungsgemäßen Mitteln vorzugsweise in einer Menge von 1 bis 50 Gew.-%, insbesondere in einer Menge von 10 bis 45 Gew.-%, besonders bevorzugt in einer Menge von 20 bis 40 Gew.-%, eingesetzt. Sorbitol wird in erfindungsgemäßen Mitteln vorzugsweise in einer Menge von 1 bis 50 Gew.-%, insbesondere in einer Menge von 10 bis 45 Gew.-%, besonders bevorzugt in einer Menge von 20 bis 40 Gew.-%, eingesetzt.

Alternativ kann eine Mischung aus Glycerin und 1 ,2-Propylenglykol eingesetzt werden. Das Glycerin wird hierbei vorzugsweise in einer Menge von 0,1 bis 50 Gew.-%, insbesondere in einer Menge von 15 bis 45 Gew.-%, besonders bevorzugt in einer Menge von 20 bis 40 Gew.-%, eingesetzt. Das 1,2-Propylenglykol wird hierbei vorzugsweise in einer Menge von 1 bis 20 Gew.-%, insbesondere in einer Menge von 5 bis 15 Gew.-%, besonders bevorzugt in einer Menge von 8 bis 12 Gew.-%, jeweils bezogen auf die Gesamtmasse des Reinigungsmittels, eingesetzt, wobei die Gesamtmenge an Glycerin und 1,2-Propylenglykol vorzugsweise mindestens 20 Gew.-%, insbesondere mindestens 25 Gew.-%, vor allem mindestens 30 Gew.-%, besonders bevorzugt 25 bis 45 Gew.-%, insbesondere 30 bis 42 Gew.-%, vor allem 35 bis 40 Gew.-% jeweils bezogen auf die Gesamtmasse des Reinigungsmittels, beträgt.

Alternativ kann eine Mischung aus Sorbitol und 1,2-Propylenglykol eingesetzt werden. Das Sorbitol wird hierbei vorzugsweise in einer Menge von 0,1 bis 50 Gew.-%, insbesondere in einer Menge von 15 bis 45 Gew.-%, besonders bevorzugt in einer Menge von 20 bis 40 Gew.-%, eingesetzt. Das 1,2-Propylenglykol wird hierbei vorzugsweise in einer Menge von 1 bis 20 Gew.-%, insbesondere in einer Menge von 5 bis 15 Gew.-%, besonders bevorzugt in einer Menge von 8 bis 12 Gew.-%, jeweils bezogen auf die Gesamtmasse des Reinigungsmittels, eingesetzt, wobei die Gesamtmenge an Sorbitol und 1,2-Propylenglykol vorzugsweise mindestens 20 Gew.-%, insbesondere mindestens 25 Gew.-%, vor allem mindestens 30 Gew.-%, besonders bevorzugt 25 bis 45 Gew.-%, insbesondere 30 bis 42 Gew.-%, vor allem 35 bis 40 Gew.-% jeweils bezogen auf die Gesamtmasse des Reinigungsmittels, beträgt.

In verschiedenen Ausführungsformen der Erfindung zeichnen sich die Reinigungsmittel dadurch aus, dass das Massenverhältnis von Glycerin zu 1,2-Propylenglykol größer als 2:1 ist.

Das Reinigungsmittel kann ferner ein phosphathaltiges Reinigungsmittel, insbesondere ein phosphathaltiges maschinelles Geschirrspülmittel sein. Die Phosphatesind dabei vorzugsweise in Form von Polyphosphaten enthalten. Erfindungsgemäß verwendbare Polyphosphate sind beispielsweise Tripolyphosphate, Pyrophosphate und Metaphosphate und dabei insbesondere deren Natrium- oder Kaliumsalze. Bevorzugt werden Tripolyphosphate eingesetzt.

Die erfindungsgemäß verwendbaren Tripolyphosphate (oder auch Triphosphate) sind Kondensationsprodukte der ortho-Phosphorsäure (H₃PO₄) mit der Summenformel P₃O₁₀⁵⁻, die gewöhnlich in Form ihrer Salze, vorzugsweise der Alkalimetall- oder Erdalkalimetall, noch bevorzugter in Form ihrer Alkalimetallsalze eingesetzt werden. Tripolyphosphatsalze sind im Allgemeinen hygroskopische, weiße, geruchlose, nicht brennbare Feststoffe, die in Wasser leicht löslich sind. Erfindungsgemäß werden besonders das Kaliumsalz von Tripolyphosphat (K₅P₃O₁₀) oder eine Mischung aus dem Kaliumsalz des Tripolyphosphats und dem Natriumsalz des Tripolyphosphats (Na₅P₃O₁₀) verwendet. Am bevorzugtesten wird ausschließlich das Kaliumsalz des Tripolyphosphats eingesetzt.

Der Gewichtsanteil der Polyphosphate, insbesondere des Tripolyphosphats, am Gesamtgewicht des erfindungsgemäßen Reinigungsmittels beträgt vorzugsweise 0,1 bis 30 Gew.-%, insbesondere von 1 bis 28 Gew.-%, besonders bevorzugt von 5 bis 25 Gew.-%, noch bevorzugter von 10 bis 23 Gew.-%.

Die Reinigungsmittel können vorzugsweise einen oder mehrere nicht-phosphathaltige Gerüststoff(e) (Builder/Cobuilder) (ggf. zusätzlich oder auch anstatt der mindestens einen phosphathaltigen Builderkomponente) enthalten. Der Gewichtsanteil dieser mindestens einen nicht-phosphathaltigen Builderkomponente zusätzlich zu der einer phosphathaltigen Builderkomponente am Gesamtgewicht der erfindungsgemäßen Mittel beträgt vorzugsweise 0,1 bis 10 Gew.-% und insbesondere 2 bis 7 Gew.-%. Der Gewichtsanteil dieser mindestens einen nicht-phosphathaltigen Builderkomponente verschiedenen Gerüststoffe am Gesamtgewicht der erfindungsgemäßen Mittel beträgt vorzugsweise 0,1 bis 60 Gew.-% und insbesondere 2 bis 45 Gew.-%. Zu diesen nicht-phosphathaltigen Builderkomponenten/Gerüststoffen zählen insbesondere Carbonate, Citrate, Phosphonate, MGDA (Methylglycindiessigsäure) oder deren Salze, GLDA (Glutaminsäure-N,N-diessigsäure) oder deren Salze, EDDS (Ethylendiamin-N,N'-dibernsteinsäure) oder deren Salze, organische Cobuilder und Silikate.

Möglich ist beispielsweise der Einsatz von Carbonat(en) und/oder Hydrogencarbonat(en), vorzugsweise Alkalicarbonat(en), besonders bevorzugt Natriumcarbonat.

Als organische Cobuilder sind insbesondere Polycarboxylate/Polycarbonsäuren, polymere Carboxylate, Asparaginsäure, Polyacetale, Dextrine und organische Cobuilder zu nennen. Diese Stoffklassen werden nachfolgend beschrieben.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure,

Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes von Reinigungsmitteln. Insbesondere sind hierbei Citronensäure, Bernsteinsäure, Glutarsäure, Adipinsäure, Gluconsäure und beliebige Mischungen aus diesen zu nennen.

Besonders bevorzugte Reinigungsmittel enthalten als einen ihrer wesentlichen nicht-phosphathaltigen Builderkomponente Gerüststoffe Citrat, beispielsweise Natrium- oder Kaliumcitrat. Reinigungsmittel, die 1 bis 10 Gew.-%, vorzugsweise 2 bis 5 Gew.-% Citrat enthalten, sind erfindungsgemäß bevorzugt.

Als Gerüststoffe sind weiterhin polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Die Reinigungsmittel können als Gerüststoff insbesondere auch Phosphonate enthalten. Als Phosphonat-Verbindung wird vorzugsweise ein Hydroxyalkan- und/oder Aminoalkanphosphonat eingesetzt. Unter den Hydroxyalkanphosphonaten ist das 1-Hydroxyethan-1,1-diphosphonat (HEDP) von besonderer Bedeutung. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Phosphonate sind in den Mitteln vorzugsweise in Mengen von 0,1 bis 10 Gew.-%, insbesondere in Mengen von 0,5 bis 8 Gew.-%, jeweils bezogen auf das Gesamtgewicht des Reinigungsmittels, enthalten.

Die Reinigungsmittel können als weitere Builderkomponente Gerüststoff weiterhin kristalline schichtförmige Silikate der allgemeinen Formel NaMSiₓO₂ₓ₊₁ · y H₂O, worin M Natrium oder Wasserstoff darstellt, x eine Zahl von 1,9 bis 22, vorzugsweise von 1,9 bis 4, wobei besonders bevorzugte Werte für x 2, 3 oder 4 sind, und y für eine Zahl von 0 bis 33, vorzugsweise von 0 bis 20 steht. Einsetzbar sind auch amorphe Natriumsilikate mit einem Modul Na₂O:SiO₂ von 1:2 bis 1:3,3, vorzugsweise von 1:2 bis 1:2,8 und insbesondere von 1:2 bis 1:2,6, welche vorzugsweise löseverzögert sind und Sekundärwascheigenschaften aufweisen.

In bevorzugten Reinigungsmitteln wird der Gehalt an Silikaten, bezogen auf das Gesamtgewicht des Reinigungsmittels, auf Mengen unterhalb 10 Gew.-%, vorzugsweise unterhalb 5 Gew.-% und insbesondere unterhalb 2 Gew.-% begrenzt. Besonders bevorzugte Reinigungsmittel sind Silikatfrei.

Die erfindungsgemäßen Reinigungsmittel können ferner ein Sulfopolymer enthalten. Der Gewichtsanteil des Sulfopolymers am Gesamtgewicht des erfindungsgemäßen Reinigungsmittels beträgt vorzugsweise von 0,1 bis 20 Gew.-%, insbesondere von 0,5 bis 18 Gew.-%, besonders bevorzugt 1,0 bis 15 Gew.-%, insbesondere von 4 bis 14 Gew.-%, vor allem von 6 bis 12 Gew.-%. Das Sulfopolymer wird üblicheweise in Form einer wässrigen Lösung eingesetzt, wobei die wässrigen Lösungen typischerweise 20 bis 70 Gew.-%, insbesondere 30 bis 50 Gew.-%, vorzugsweise etwa 35 bis 40 Gew.-% Sulfopolymer(e) enthalten.

Als Sulfopolymer wird vorzugsweise ein copolymeres Polysulfonat, vorzugsweise ein hydrophob modifiziertes copolymeres Polysulfonat, eingesetzt.

Die Copolymere können zwei, drei, vier oder mehr unterschiedliche Monomereinheiten aufweisen.

Bevorzugte copolymere Polysulfonate enthalten neben Sulfonsäuregruppen-haltigem(n) Monomer(en) wenigstens ein Monomer aus der Gruppe der ungesättigten Carbonsäuren.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettiger oder verzweigter Kohlenwasserstoffrest mit 1 bis 12 Kohlenstoffatomen ist.

Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren.

Bei den Sulfonsäuregruppen-haltigen Monomeren sind solche der Formel R⁵(R⁶)C=C(R⁷)-X-SO₃H bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste oder für -COOH oder -COOR⁴ steht, wobei R⁴ ein gesättigter oder ungesättigter, geradkettiger oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₃)-CH₂-.

Unter diesen Monomeren bevorzugt sind solche der Formeln

H₂C=CH-X-SO₃H

H₂C=C(CH₃)-X-SO₃H

HO₃S-X-(R⁶)C=C(R⁷)-X-SO₃H,

in denen R⁶ und R⁷ unabhängig voneinander ausgewählt sind
aus -H, -CH₃, -CH₂CH₃, -CH₂CH₂CH₃ und -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₃)-CH₂-.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze.

In den Polymeren können die Sulfonsäuregruppen ganz oder teilweise in neutralisierter Form vorliegen, d.h. dass das acide Wasserstoffatom der Sulfonsäuregruppe in einigen oder allen Sulfonsäuregruppen gegen Metallionen, vorzugsweise Alkalimetallionen und insbesondere gegen Natriumionen, ausgetauscht sein kann. Der Einsatz von teil- oder vollneutralisierten sulfonsäuregruppenhaltigen Copolymeren ist erfindungsgemäß bevorzugt.

Die Monomerenverteilung der erfindungsgemäß bevorzugt eingesetzten Copolymere beträgt bei Copolymeren, die nur Carbonsäuregruppen-haltige Monomere und Sulfonsäuregruppen-haltige Monomere enthalten, vorzugsweise jeweils 5 bis 95 Gew.-%, besonders bevorzugt beträgt der Anteil des Sulfonsäuregruppen-haltigen Monomers 50 bis 90 Gew.-% und der Anteil des Carbonsäuregruppen-haltigen Monomers 10 bis 50 Gew.-%, die Monomere sind hierbei vorzugsweise ausgewählt aus den zuvor genannten.

Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte Reinigungsmittel sind dadurch gekennzeichnet, dass die Copolymere Molmassen von 2.000 bis 200.000 gmol⁻¹, vorzugsweise von 4.000 bis 25.000 gmol⁻¹ und insbesondere von 5.000 bis 15.000 gmol⁻¹ aufweisen.

In einer weiteren bevorzugten Ausführungsform umfassen die Copolymere neben Carboxylgruppen-haltigem Monomer und Sulfonsäuregruppen-haltigem Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer maschineller Geschirrspülmittel verbessert werden.

Anionische Copolymere umfassend Carbonsäuregruppen-haltige Monomere, Sulfonsäuregruppen-haltige Monomere und nichtionische Monomere, insbesondere hydrophobe Monomere, werden daher erfindungsgemäß bevorzugt.

Als nichtionische Monomere werden vorzugsweise Monomere der allgemeinen Formel R¹(R²)C=C(R³)-X-R⁴ eingesetzt, in der R¹ bis R³ unabhängig voneinander für -H, -CH₃ oder -C₂H₅ steht, X für eine optional vorhandene Spacergruppe steht, die ausgewählt ist aus -CH₂-, -C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht.

Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2, 2,3-Dimethylhexen-1, 2,4-Dimethylhexen-1, 2,5-Dimethlyhexen-1, 3,5-Dimethylhexen-1, 4,4-Dimehtylhexan-1, Ethylcyclohexyn, 1-Octen, α-Olefine mit 10 oder mehr Kohlenstoffatomen wie beispielsweise 1-Decen, 1-Dodecen, 1-Hexadecen, 1-Oktadecen und C₂₂-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2-Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, N-(Methyl)acrylamid, Acrylsäure-2-Ethylhexylester, Methacrylsäure-2-Ethylhexylester, *N*-(2-Ethylhexyl)acrylamid, Acrylsäureoctylester, Methacrylsäureoctylester, *N*-(Octyl)acrylamid, Acrylsäurelaurylester, Methacrylsäurelaurylester, *N*-(Lauryl)acrylamid, Acrylsäurestearylester, Methacrylsäurestearylester, *N*-(Stearyl)acrylamid, Acrylsäurebehenylester, Methacrylsäurebehenylester und *N*-(Behenyl)acrylamid oder deren Mischungen.

Die Monomerenverteilung der erfindungsgemäß bevorzugt eingesetzten hydrophob modifizierten Copolymere beträgt in Bezug auf das Sulfonsäuregruppen-haltige Monomer, das hydrophobe Monomer und das Carbonsäuregruppen-haltige Monomer vorzugsweise jeweils 5 bis 80 Gew.-%, besonders bevorzugt beträgt der Anteil des Sulfonsäuregruppen-haltigen Monomers und des hydrophoben Monomers jeweils 5 bis 30 Gew.-% und der Anteil des Carbonsäuregruppen-haltigen Monomers 60 bis 80 Gew.-%, die Monomere sind hierbei vorzugsweise ausgewählt aus den zuvor genannten.

In Ergänzung zu den vorgenannten Gerüststoffen können die Reinigungsmittel Alkalimetallhydroxide enthalten. Diese Alkaliträger werden in den Reinigungsmitteln bevorzugt nur in geringen Mengen, vorzugsweise in Mengen unterhalb 10 Gew.-%, bevorzugt unterhalb 6 Gew.-%, vorzugsweise unterhalb 5 Gew.-% jeweils bezogen auf das Gesamtgewicht des Reinigungsmittels eingesetzt. Bevorzugte erfindungsgemäße Reinigungsmittel sind frei von Alkalimetallhydroxiden.

Die erfindungsgemäßen Reinigungsmittel enthalten vorzugsweise weiterhin mindestens ein nichtionisches Tensid. Als nichtionische Tenside können alle dem Fachmann bekannten nichtionischen Tenside eingesetzt werden. Bevorzugt werden schwachschäumende nichtionische Tenside eingesetzt, insbesondere alkoxylierte, vor allem ethoxylierte, schwachschäumende nichtionische Tenside. Mit besonderem Vorzug enthalten die maschinellen Geschirrspülmittel nichtionische Tenside aus der Gruppe der alkoxylierten Alkohole.

Insbesondere bevorzugt sind nichtionische Tenside, die einen Schmelzpunkt oberhalb Raumtemperatur aufweisen. Nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, ist/sind besonders bevorzugt.

Bevorzugt einzusetzende Tenside stammen aus den Gruppen der alkoxylierten Niotenside, insbesondere der ethoxylierten primären Alkohole und Mischungen dieser Tenside mit strukturell komplizierter aufgebauten Tensiden wie Polyoxypropylen/Polyoxyethylen/Polyoxypropylen ((PO/EO/PO)-Tenside). Solche (PO/EO/PO)-Niotenside zeichnen sich darüber hinaus durch gute Schaumkontrolle aus.

Als besonders bevorzugte Niotenside haben sich im Rahmen der vorliegenden Erfindung schwachschäumende Niotenside erwiesen, welche alternierende Ethylenoxid- und Alkylenoxideinheiten aufweisen. Unter diesen sind wiederum Tenside mit EO-AO-EO-AO-Blöcken bevorzugt, wobei jeweils eine bis zehn EO- bzw. AO-Gruppen aneinandergebunden sind, bevor ein Block aus den jeweils anderen Gruppen folgt. Hier sind nichtionische Tenside der allgemeinen Formel 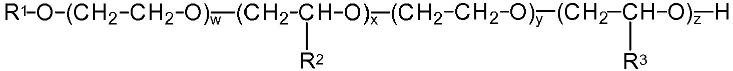 bevorzugt, in der R¹ für einen geradkettigen oder verzweigten, gesättigten oder ein- bzw. mehrfach ungesättigten C₆₋₂₄-Alkyl- oder -Alkenylrest steht; jede Gruppe R² bzw. R³ unabhängig voneinander ausgewählt ist aus -CH₃, -CH₂CH₃, -CH₂CH₂-CH₃, CH(CH₃)₂ und die Indizes w, x, y, z unabhängig voneinander für ganze Zahlen von 1 bis 6 stehen.

Somit sind insbesondere nichtionische Tenside bevorzugt, die einen C₉₋₁₅-Alkylrest mit 1 bis 4 Ethylenoxideinheiten, gefolgt von 1 bis 4 Propylenoxideinheiten, gefolgt von 1 bis 4 Ethylenoxideinheiten, gefolgt von 1 bis 4 Propylenoxideinheiten aufweisen.

Bevorzugte nichtionische Tenside sind hierbei solche der allgemeinen Formel R¹-CH(OH)CH₂O-(AO)_{w}-(A'O)ₓ-(A"O)_{y}-(A‴O)_{z}-R², in der
- R¹ für einen geradkettigen oder verzweigten, gesättigten oder ein- bzw. mehrfach ungesättigten C₆₋₂₄-Alkyl- oder -Alkenylrest steht;
- R² für H oder einen linearen oder verzweigten Kohlenwasserstoffrest mit 2 bis 26 Kohlenstoffatomen steht;
- A, A', A" und A‴ unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) stehen,
- w, x, y und z für Werte zwischen 0,5 und 120 stehen, wobei x, y und/oder z auch 0 sein können.

Durch den Zusatz der vorgenannten nichtionischen Tenside der allgemeinen Formel R¹-CH(OH)CH₂O-(AO)_{w}-(A'O)ₓ-(A"O)_{y}-(A‴O)_{z}-R², nachfolgend auch als "Hydroxymischether" bezeichnet, kann die Reinigungsleistung der erfindungsgemäßen Zubereitungen deutlich verbessert werden und zwar sowohl im Vergleich zu Tensid-freien System wie auch im Vergleich zu Systemen, die alternative nichtionischen Tenside, beispielsweise aus der Gruppe der polyalkoxylierten Fettalkohole enthalten.

Bevorzugt werden insbesondere solche endgruppenverschlossene, poly(oxyalkylierten) Niotenside, die, gemäß der Formel R¹O[CH₂CH₂O]ₓCH₂CH(OH)R², neben einem Rest R¹, welcher für lineare oder verzweigte, gesättigte oder ungesättigte, aliphatische oder aromatische Kohlenwasserstoffreste mit 2 bis 30 Kohlenstoffatomen, vorzugsweise mit 4 bis 22 Kohlenstoffatomen steht, weiterhin einen linearen oder verzweigten, gesättigten oder ungesättigten, aliphatischen oder aromatischen Kohlenwasserstoffrest R² mit 1 bis 30 Kohlenstoffatomen aufweisen, wobei x für Werte zwischen 1 und 90, vorzugsweise für Werte zwischen 30 und 80 und insbesondere für Werte zwischen 30 und 60 steht.

Besonders bevorzugt sind Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 sowie y für einen Wert von mindestens 15 steht.

Zur Gruppe dieser nichtionischen Tenside zählen beispielsweise die C₂₋₂₆ Fettalkohol-(PO)₁-(EO)₁₅₋₄₀-2-hydroxyalkylether, insbesondere auch die C₈₋₁₀ Fettalkohol-(PO)₁-(EO)₂₂-2-hydroxydecylether.

Besonders bevorzugt werden weiterhin solche endgruppenverschlossene poly(oxyalkylierten) Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für -CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel R¹O[CH₂CH(R³)O]ₓ[CH₂]ₖCH(OH)[CH₂]ⱼOR², in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der oben stehenden Formel R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der obenstehenden Formel unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Besonders bevorzugte endgruppenverschlossene poly(oxyalkylierte) Alkohole der oben stehenden Formel weisen Werte von k = 1 und j = 1 auf, so dass sich die vorstehende Formel zu R¹O[CH₂CH(R³)O]ₓCH₂CH(OH)CH₂OR² vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Als besonders wirkungsvoll haben sich schließlich die nichtionischen Tenside der allgemeine Formel R¹-CH(OH)CH₂O-(AO)_{w}-R² erwiesen, in der
- R¹ für einen geradkettigen oder verzweigten, gesättigten oder ein- bzw. mehrfach ungesättigten C₆₋₂₄-Alkyl- oder -Alkenylrest steht;
- R² für einen linearen oder verzweigten Kohlenwasserstoffrest mit 2 bis 26 Kohlenstoffatomen steht;
- A für einen Rest aus der Gruppe CH₂CH₂, CH₂CH₂CH₂, CH₂CH(CH₃), vorzugsweise für CH₂CH₂ steht, und
- w für Werte zwischen 1 und 120, vorzugsweise 10 bis 80, insbesondere 20 bis 40 steht.

Zur Gruppe dieser nichtionischen Tenside zählen beispielsweise die C₄₋₂₂ Fettalkohol-(EO)₁₀₋₈₀-2-hydroxyalkylether, insbesondere auch die C₈₋₁₂ Fettalkohol-(EO)₂₂-2-hydroxydecylether und die C₄-₂₂ Fettalkohol-(EO)₄₀₋₈₀-2-hydroxyalkylether.

In verschiedenen Ausführungsformen der Erfindung können anstelle der oben definierten endgruppenverschlossenen Hydroxymischether auch die entsprechenden nicht endgruppenverschlossenen Hydroxymischether eingesetzt werden. Diese können den obigen Formeln genügen, wobei R² aber Wasserstoff ist und R¹, R³, A, A', A", A‴, w, x, y und z wie oben definiert sind.

Bevorzugte flüssige Reinigungsmittel sind dadurch gekennzeichnet, dass das Reinigungsmittel mindestens ein nichtionisches Tensid, vorzugsweise ein nichtionisches Tensid aus der Gruppe der Hydroxymischether, enthält, wobei der Gewichtsanteil des nichtionischen Tensids am Gesamtgewicht des Reinigungsmittels vorzugsweise 0,1 bis 10 Gew.-%, bevorzugt 0,5 bis 8,0 Gew.-% und insbesondere 1,0 bis 4,0 Gew.-% beträgt.

Generell kann der pH-Wert des Reinigungsmittels mittels üblicher pH-Regulatoren eingestellt werden, wobei der pH-Wert abhängig von dem gewünschten Einsatzzweck gewählt wird. In verschiedenen Ausführungsformen liegt der pH-Wert in einem Bereich von 5,5 bis 10,5, vorzugsweise 5,5 bis 9,5, noch bevorzugter 7 bis 9, insbesondere größer 7, vor allem im Bereich 7,5 bis 8,5. Als pH-Stellmittel dienen Säuren und/oder Alkalien, vorzugsweise Alkalien. Geeignete Säuren sind insbesondere organische Säuren wie die Essigsäure, Zitronensäure, Glycolsäure, Milchsäure, Bernsteinsäure, Adipinsäure, Äpfelsäure, Weinsäure und Gluconsäure oder auch Amidosulfonsäure. Daneben können aber auch die Mineralsäuren Salzsäure, Schwefelsäure und Salpetersäure bzw. deren Mischungen eingesetzt werden. Geeignete Basen stammen aus der Gruppe der Alkali- und Erdalkalimetallhydroxide und -carbonate, insbesondere der Alkalimetallhydroxide, von denen Kaliumhydroxid und vor allem Natriumhydroxid bevorzugt ist. Besonders bevorzugt ist allerdings flüchtiges Alkali, beispielsweise in Form von Ammoniak und/oder Alkanolaminen, die bis zu 9 C-Atome im Molekül enthalten können. Das Alkanolamin ist hierbei vorzugsweise ausgewählt aus der Gruppe bestehend aus Mono-, Di-, Triethanol- und -Propanolamin und deren Mischungen. Das Alkanolamin ist in erfindungsgemäßen Mitteln vorzugsweise in einer Menge von 0,5 bis 10 Gew.-%, insbesondere in einer Menge von 1 bis 6 Gew.-%, enthalten.

Zur Einstellung und/oder Stabilisierung des pH-Werts kann das erfindungsgemäße Mittel ein oder mehrere Puffersubstanzen (INCI Buffering Agents) enthalten, üblicherweise in Mengen von 0,001 bis 5 Gew.-%. Bevorzugt sind Puffersubstanzen, die zugleich Komplexbildner oder sogar Chelatbildner (Chelatoren, INCI Chelating Agents) sind. Besonders bevorzugte Puffersubstanzen sind die Citronensäure bzw. die Citrate, insbesondere die Natrium- und Kaliumcitrate, beispielsweise Trinatriumcitrat·2H₂O und Trikaliumcitrat·H₂O.

Die erfindungsgemäßen Mittel enthalten vorzugsweise mindestens einen weiteren Bestandteil, vorzugsweise ausgewählt aus der Gruppe bestehend aus anionischen, kationischen und amphoteren Tensiden, Bleichmitteln, Bleichaktivatoren, Bleichkatalysatoren, Polymeren, Nicht-Amylase- und Nicht-Protease-Enzymen, Verdickern, Sequestrierungsmitteln, Elektrolyten, Korrosionsinhibitoren, insbesondere Silberschutzmitteln, Glaskorrosionsinhibitoren, Schauminhibitoren, Farbstoffen, Duftstoffen, Bitterstoffen, und antimikrobiellen Wirkstoffen.

Bevorzugte anionische Tenside sind Fettalkoholsulfate, Fettalkoholethersulfate, Dialkylethersulfate, Monoglyceridsulfate, Alkylbenzolsulfonate, Olefinsulfonate, Alkansulfonate, Ethersulfonate, n-Alkylethersulfonate, Estersulfonate und Ligninsulfonate. Ebenfalls im Rahmen der vorliegenden Erfindung verwendbar sind Fettsäurecyanamide, Sulfosuccinate (Sulfobernsteinsäureester), insbesondere Sulfobernsteinsäuremono- und -di-C₈-C₁₈-Alkylester, Sulfosuccinamate, Sulfosuccinamide, Fettsäureisethionate, Acylaminoalkansulfonate (Fettsäuretauride), Fettsäuresarcosinate, Ethercarbonsäuren und Alkyl(ether)phosphate sowie α-Sulfofettsäuresalze, Acylglutamate, Monoglyceriddisulfate und Alkylether des Glycerindisulfats.

Die anionischen Tenside werden vorzugsweise als Natriumsalze eingesetzt, können aber auch als andere Alkali- oder Erdalkalimetallsalze, beispielsweise Kalium- oder Magnesiumsalze, sowie in Form von Ammonium- oder Mono-, Di-, Tri- bzw. Tetraalkylammoniumsalzen enthalten sein, im Falle der Sulfonate auch in Form ihrer korrespondierenden Säure, z.B. Dodecylbenzolsulfonsäure.

Geeignete Amphotenside sind beispielsweise Betaine der Formel (Rⁱⁱⁱ)(R^{iv})(R^{v})N⁺CH₂COO⁻, in der Rⁱⁱⁱ einen gegebenenfalls durch Heteroatome oder Heteroatomgruppen unterbrochenen Alkylrest mit 8 bis 25, vorzugsweise 10 bis 21 Kohlenstoffatomen und R^{iv} sowie R^{v} gleichartige oder verschiedene Alkylreste mit 1 bis 3 Kohlenstoffatomen bedeuten, insbesondere C₁₀-C₁₈-Alkyldimethylcarboxymethylbetain und C₁₁-C₁₇-Alkylamidopropyl-dimethylcarboxymethylbetain.

Geeignete Kationtenside sind u.a. die quartären Ammoniumverbindungen der Formel (R^{vi})(R^{vii})(R^{viii})(R^{ix})N⁺ X⁻, in der R^{vi} bis R^{ix} für vier gleich- oder verschiedenartige, insbesondere zwei lang- und zwei kurzkettige, Alkylreste und X⁻ für ein Anion, insbesondere ein Halogenidion, stehen, beispielsweise Didecyl-dimethyl-ammoniumchlorid, Alkyl-benzyl-didecyl-ammoniumchlorid und deren Mischungen. Weitere geeignete kationische Tenside sind die quaternären oberflächenaktiven Verbindungen, insbesondere mit einer Sulfonium-, Phosphonium-, Jodonium- oder Arsoniumgruppe, die auch als antimikrobielle Wirkstoffe bekannt sind. Durch den Einsatz von quaternären oberflächenaktiven Verbindungen mit antimikrobieller Wirkung kann das Mittel mit einer antimikrobiellen Wirkung ausgestaltet werden bzw. dessen gegebenenfalls aufgrund anderer Inhaltsstoffe bereits vorhandene antimikrobielle Wirkung verbessert werden.

Zu den Enzymen gehören neben den oben genannten Proteasen und Amylasen insbesondere Lipasen, Hemicellulasen, Cellulasen, Perhydrolasen und Oxidoreduktasen, sowie vorzugsweise deren Gemische. Diese Enzyme sind im Prinzip natürlichen Ursprungs; ausgehend von den natürlichen Molekülen stehen für den Einsatz in Reinigungsmitteln verbesserte Varianten zur Verfügung, die entsprechend bevorzugt eingesetzt werden. Erfindungsgemäße Reinigungsmittel enthalten diese Enzyme vorzugsweise in Gesamtmengen von 1 × 10⁻⁶ bis 5 Gew.-% bezogen auf aktives Protein.

Erfindungsgemäß einsetzbar sind Lipasen oder Cutinasen, insbesondere wegen ihrer Triglyceridspaltenden Aktivitäten, aber auch, um aus geeigneten Vorstufen *in situ* Persäuren zu erzeugen. Hierzu gehören beispielsweise die ursprünglich aus *Humicola lanuginosa* (*Thermomyces lanuginosus*) erhältlichen, beziehungsweise weiterentwickelten Lipasen, insbesondere solche mit dem Aminosäureaustausch D96L.

Weiterhin können Enzyme eingesetzt werden, die unter dem Begriff Hemicellulasen zusammengefasst werden. Hierzu gehören beispielsweise Mannanasen, Xanthanlyasen, Pektinlyasen (=Pektinasen), Pektinesterasen, Pektatlyasen, Xyloglucanasen (= Xylanasen), Pullulanasen und β-Glucanasen.

Zur Erhöhung der bleichenden Wirkung können erfindungsgemäß Oxidoreduktasen, beispielsweise Oxidasen, Oxygenasen, Katalasen, Peroxidasen, wie Halo-, Chloro-, Bromo-, Lignin-, Glucose- oder Manganperoxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Die Enzyme können konfektioniert und formuliert sein wie bereits oben für die die Proteasen und Amylasen beschrieben.

Als Glaskorrosionsinhibitoren werden vorzugsweise Zinksalze, insbesondere Zinkacetat, eingesetzt. Glaskorrosionsinhibitoren sind in erfindungsgemäßen Mitteln vorzugsweise in einer Menge von 0,05 bis 5 Gew.-%, insbesondere in einer Menge von 0,1 bis 2 Gew.-%, enthalten.

In verschiedenen Ausführungsformen besitzt das Reinigungsmittel direkt nach der Herstellung eine Viskosität oberhalb 2.000 mPas (Brookfield Viscometer DV-II+Pro, Spindel 25, 30 rpm, 20°C), insbesondere zwischen 2.000 und 10.000 mPas. Nach der Lagerung kann die Viskosität höher sein, beispielsweise größer als 10.000 mPas, wie zum Beispiel im Bereich 10.000-50.000 mPas, vorzugsweise um 35.000 mPas (Brookfield Viscometer DV-ll+Pro, Spindel 25, 5 rpm, 20°C).

Das Reinigungsmittel kann sich in einer wasserunlöslichen, wasserlöslichen oder wasserdispergierbaren Verpackung befinden. Die Erfindung betrifft daher auch Kits die das Reinigungsmittel zusammen mit einer solchen Verpackung enthalten. Das Reinigungsmittel kann dabei derart konfektioniert sein, dass Einmalportionen jeweils separat verpackt sind.

Vorzugsweise ist das erfindungsgemäße Reinigungsmittel in einer wasserlöslichen Verpackung enthalten. Die wasserlösliche Verpackung erlaubt eine Portionierung des Reinigungsmittels. Die Menge an Reinigungsmittel in der Portionspackung beträgt vorzugsweise 5 bis 50 g, besonders bevorzugt 10 bis 30 g, vor allem 15 bis 25 g.

Bei den wasserlöslichen Umhüllungen/Verpackungen handelt es sich vorzugsweise um Tiefziehkörper oder Spritzgusskörper.

Die wasserlöslichen Behälter/Umhüllungen/Verpackungen können auch durch Spritzgießen hergestellt werden. Spritzgießen bezeichnet dabei das Umformen einer Formmasse derart, dass die in einem Massezylinder für mehr als einen Spritzgießvorgang enthaltene Masse unter Wärmeeinwirkung plastisch erweicht und unter Druck durch eine Düse in den Hohlraum eines vorher geschlossenen Werkzeuges einfließt. Das Verfahren wird hauptsächlich bei nichthärtbaren Formmassen angewendet, die im Werkzeug durch Abkühlen erstarren. Der Spritzguß ist ein sehr wirtschaftliches modernes Verfahren zur Herstellung spanlos geformter Gegenstände und eignet sich besonders für die automatisierte Massenfertigung. Im praktischen Betrieb erwärmt man die thermoplastische Formmassen (Pulver, Körner, Würfel, Pasten u. a.) bis zur Verflüssigung (bis 180°C) und spritzt sie dann unter hohem Druck (bis 140 MPa) in geschlossene, zweiteilige, das heißt aus Gesenk (früher Matrize) und Kern (früher Patrize) bestehende, vorzugsweise wassergekühlte Hohlformen, wo sie abkühlen und erstarren. Einsetzbar sind Kolben- und Schneckenspritzgussmaschinen.

Solche Formkörper können ebenfalls ein, zwei, drei oder mehrere Kammern aufweisen und mit flüssigen und/oder festen Zusammensetzungen befüllt sein, von denen eine der Zusammensetzungen eine der erfindungsgemäßen Zusammensetzungen ist. Es ist beispielsweise möglich, die Kammern auf der offenen Seite entweder mit einem zweiten Formgusskörper oder mit einer oder mehreren wasserlöslichen Folien (insbesondere wie hierin beschrieben) zu verschließen. So kann die Freisetzung der in den Kammern befindlichen Zusammensetzungen beliebig nach dem gewünschten Freisetzungszeitpunkt gesteuert werden. Entweder kann das gesamte Mittel auf einmal (entweder direkt zu Beginn des Reinigungszyklus oder zu einem bestimmten Zeitpunkt im Verlauf des Reinigungszyklus) oder durch eine Variation der Folienzusammensetzung zu bestimmten, voneinander verschiedenen Zeitpunkten innerhalb des Zyklus der Geschirrspülmaschine (beispielsweise in Abhängigkeit von der Temperatur des Spülwassers) freigesetzt werden.

Die wasserlösliche Umhüllung wird vorzugsweise aus einem wasserlöslichen Folienmaterial, welches ausgewählt ist aus der Gruppe, bestehend aus Polymeren oder Polymergemischen, gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein. Besonders bevorzugt sind Folien, die beispielsweise zu Verpackungen wie Schläuchen oder Kissen verklebt und/oder versiegelt werden können, nachdem sie mit einem Mittel befüllt wurden.

Es ist bevorzugt, dass die wasserlösliche Umhüllung Polyvinylalkohol oder ein Polyvinylalkoholcopolymer enthält. Wasserlösliche Umhüllungen, die Polyvinylalkohol oder ein Polyvinylalkoholcopolymer enthalten, weisen eine gute Stabilität bei einer ausreichend hohen Wasserlöslichkeit, insbesondere Kaltwasserlöslichkeit, auf.

Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10.000 bis 1.000.000 gmol⁻¹, vorzugsweise von 20.000 bis 500.000 gmol⁻¹, besonders bevorzugt von 30.000 bis 100.000 gmol⁻¹ und insbesondere von 40.000 bis 80.000 gmol⁻¹ liegt.

Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% ausmacht.

Einem zur Herstellung der wasserlöslichen Umhüllung geeignetem Polyvinylalkohol-enthaltendem Folienmaterial kann zusätzlich ein Polymer ausgewählt aus der Gruppe umfassend (Meth)Acrylsäure-haltige (Co)Polymere, Polyacrylamide, Oxazolin-Polymere, Polystyrolsulfonate, Polyurethane, Polyester, Polyether, Polymilchsäure oder Mischungen der vorstehenden Polymere zugesetzt sein. Ein bevorzugtes zusätzliches Polymer sind Polymilchsäuren.

Bevorzugte Polyvinylalkoholcopolymere umfassen neben Vinylalkohol Dicarbonsäuren als weitere Monomere. Geeignete Dicarbonsäure sind Itaconsäure, Malonsäure, Bernsteinsäure und Mischungen daraus, wobei Itaconsäure bevorzugt ist.

Ebenfalls bevorzugte Polyvinylalkoholcopolymere umfassen neben Vinylalkohol eine ethylenisch ungesättige Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Es kann bevorzugt sein, dass das Folienmaterial weitere Zusatzstoffe enthält. Das Folienmaterial kann beispielsweise Weichmacher wie Dipropylenglycol, Ethylenglycol, Diethylenglycol, Propylenglycol, Glycerin, Sorbitol, Mannitol oder Mischungen daraus enthalten. Weitere Zusatzstoffe umfassen beispielsweise Freisetzungshilfen, Füllmittel, Vernetzungsmittel, Tenside, Antioxidationsmittel, UV-Absorber, Antiblockmittel, Antiklebemittel oder Mischungen daraus.

Geeignete wasserlösliche Folien zum Einsatz in den wasserlöslichen Umhüllungen der wasserlöslichen Verpackungen gemäß der Erfindung sind Folien, die von der Firma MonoSol LLC beispielsweise unter der Bezeichnung M8630, C8400 oder M8900 vertrieben werden. Andere geeignete Folien umfassen Folien mit der Bezeichnung Solublon^{®} PT, Solublon^{®} GA, Solublon^{®} KC oder Solublon^{®} KL von der Aicello Chemical Europe GmbH oder die Folien VF-HP von Kuraray.

Die erfindungsgemäßen Reinigungsmittel können als Geschirrspülmittel, insbesondere maschinelle Geschirrspülmittel verwendet werden. Die entsprechende Verwendung ist ebenfalls Gegenstand der Erfindung. Ebenso betrifft die Erfindung ein Geschirrspülverfahren, insbesondere maschinelles Geschirrspülverfahren, bei welchem ein Reinigungsmittel gemäß der Erfindung eingesetzt wird.

Gegenstand der vorliegenden Anmeldung ist daher weiterhin ein Verfahren zur Reinigung von Geschirr in einer Geschirrspülmaschine, bei welchem das Mittel während des Durchlaufens eines Geschirrspülprogramms vor Beginn des Hauptspülgangs oder im Verlaufe des Hauptspülgangs in den Innenraum einer Geschirrspülmaschine eindosiert wird. Die Eindosierung bzw. der Eintrag des Mittels in den Innenraum der Geschirrspülmaschine kann manuell erfolgen, vorzugsweise wird das Mittel jedoch mittels der Dosierkammer in den Innenraum der Geschirrspülmaschine dosiert. In verschiedenen Ausführungsformen der Erfindung liegt bei solchen Geschirrspülverfahren die (Spül-)Temperatur vorzugsweise bei 50°C oder niedriger, besonders bevorzugt 45°C oder niedriger, noch bevorzugter 40°C oder niedriger.

Alle im Zusammenhang mit den hierin offenbarten Reinigungsmitteln als besondere Ausführungsformen beschriebenen Enzymkombinationen sind ebenso in den beschriebenen Verfahren und Verwendungen einsetzbar.

### Beispiel 1: Flüssige Geschirrspülmittelformulierunqen enthaltend Proteasen

**Tabelle 1: Zusammensetzung des maschinellen Geschirrspülmittels (Angaben in Gew.-% Aktivsubstanz)**

| | Rezeptur 1 | Rezeptur 2 |
|---|---|---|
| Glycerin | 26 | 26 |
| 1,2-Propylenglykol | 10,00 | 10,00 |
| Sulfopolymer | 8,5 | 8,5 |
| Kaliumtripolyphosphat | 21 | 21 |
| Na Citrat Dihydrat | 4,00 | 4,00 |
| HEDP | 2,40 | 2,40 |
| Niotensid | 2,00 | 2,00 |
| Ethanolamin | 3,50 | 3,50 |
| Polyacrylat | 0,20 | 0,20 |
| Protease 2 | 0,00 | 0,20 |
| Amylase 2 | 0,015 | 0,015 |
| Protease 1 | 0,3 | 0,1 |
| Amylase 1 | 0,00 | 0,00 |
| Zinkacetat | 0,20 | 0,20 |
| Bitterstoff, Konservierungsmittel, Farbstoff, Parfum, Wasser | Ad 100 | Ad 100 |

Angaben beziehen sich auf den Gewichtsanteil an dem jeweiligen Aktivprotein
Protease1 = fest, SEQ ID NO:8
Protease 2 = flüssig, SEQID NO:9
Amylase 1 = fest, Variante der α-Amylase aus *Bacillus sp.* No. 707
Amylase 2 = flüssig, Stainzyme^{®} 12L (Novozymes, DK)

### Reinigungsleistung an proteasesensitiven Eigelb-Anschmutzungen

In einer Miele GSL Geschirrspülmaschine wurde bei 50°C (Programm "Normal") und 21°dH Geschirr mit einer Anschmutzung aus Eigelb mit einer flüssigen Geschirrspülmittelzusammensetzung (Zusammensetzung siehe Tabelle 1: Rezeptur 1 = Vergleichsrezeptur, Rezeptur 2 = erfindungsgemäß) gespült. Nach jedem Spülzyklus wurde die Reinigungsleistung gravimetrisch bestimmt (Auswertung von 1-10, je höher der Wert, desto besser die Leistung, Unterschiede von mindestens 1 sind signifikant). Die Ergebnisse für die getesteten Rezepturen sind in der Tabelle 2 als arithmetische Mittelwerte aufgelistet. Höhere Werte bedeuten eine bessere Reinigungsleistung.

**Tabelle 2: Reinigungsleistung an Eigelb**

| | Eigelb |
|---|---|
| Rezeptur 1 | 2,6 |
| Rezeptur 2 | 3,5 |

Der Tabelle 2 ist eindeutig zu entnehmen, dass die Kombination der zwei verschiedenen Enzymformulierungen zu einer deutlichen Verbesserung der Reinigungsleistung führt, obwohl beide Rezepturen den gleichen Aktivenzymgehalt aufweisen.

### Beispiel 2: Flüssige Geschirrspülmittelformulierunqen enthaltend Amylasen

**Tabelle 3: Zusammensetzung des maschinellen Geschirrspülmittels (Angaben in Gew.-% Aktivsubstanz)**

| | Rezeptur 3 | Rezeptur 4 | Rezeptur 5 |
|---|---|---|---|
| Glycerin | 26 | 26 | 26 |
| 1,2-Propylenglykol | 10,00 | 10,00 | 10,00 |
| Sulfopolymer | 8,5 | 8,5 | 8,5 |
| Kaliumtripolyphosphat | 21 | 21 | 21 |
| Na Citrat Dihydrat | 4,00 | 4,00 | 4,00 |
| HEDP | 2,40 | 2,40 | 2,40 |
| Niotensid | 2,00 | 2,00 | 2,00 |
| Ethanolamin | 3,50 | 3,50 | 3,50 |
| Polyacrylat | 0,20 | 0,20 | 0,20 |
| Protease 2 | 0,20 | 0,20 | 0,20 |
| Amylase 2 | 0,075 | 0,00 | 0,015 |
| Protease 1 | 0,00 | 0,00 | 0,00 |
| Amylase 1 | 0,025 | 0,05 | 0,00 |
| Zinkacetat | 0,20 | 0,20 | 0,20 |
| Bitterstoff, Konservierungsmittel, Farbstoff, Parfum, Wasser | Ad 100 | Ad 100 | Ad 100 |

| | | | |
|---|---|---|---|
| Protease1 = fest, SEQ ID NO:8 Protease 2 = flüssig, SEQID NO:9 Amylase 1 = fest, Variante der α-Amylase aus *Bacillus sp.* No. 707 Amylase 2 = flüssig, Stainzyme^{®} 12L (Novozymes, DK) | | | |

In einer Miele GSL Geschirrspülmaschine wurde bei 50 °C (Programm "Normal") und 21°dH Geschirr mit einer Anschmutzung aus Haferbrei bzw. Spaghetti mit einer flüssigen Geschirrspülmittelzusammensetzung (Zusammensetzung siehe Tabelle 3: Rezepturen 4/5 = Vergleichsrezeptur, Rezeptur 3 = erfindungsgemäß) gespült. Nach jedem Spülzyklus wurde die Reinigungsleistung visuell nach IKW bestimmt (Auswertung von 1-10, je höher der Wert, desto besser die Leistung, Unterschiede von mindestens 1 sind signifikant). Die Ergebnisse für die getesteten Rezepturen sind in der Tabelle 4 als arithmetische Mittelwerte aufgelistet. Höhere Werte bedeuten eine bessere Reinigungsleistung.

**Tabelle 4: Reinigungsleistung an Haferbrei/Spaghetti**

| | Haferbrei | Spaghetti |
|---|---|---|
| Rezeptur 3 | 7,8 | 3,9 |
| Rezeptur 4 | 7,0 | 2,6 |
| Rezeptur 5 | 7,3 | 3,6 |

Tabelle 4 ist deutlich zu entnehmen, dass die erfindungsgemäß Kombination zu einer Erhöhung der Reinigungsleistung führt.

## Patentansprüche

1. Flüssiges Reinigungsmittel, insbesondere maschinelles Geschirrspülmittel, enthaltend
(1) mindestens eine flüssige Enzymformulierung, die mindestens eine Amylase (A1) und optional mindestens eine Protease (P1) enthält; und
(2) mindestens eine feste Enzymformulierung, vorzugsweise in Form eines Granulats, die mindestens eine Protease (P2) und/oder mindestens eine Amylase (A2) enthält, wobei die feste Enzymformulierung in dem flüssigen Geschirrspülmittel homogen suspendiert ist,
**dadurch gekennzeichnet, dass**
a) die Amylase (A1) eine AA560 α-Amylase aus *Bacillus sp.* oder ein funktionales Fragment davon ist; und/oder
b) die Amylase (A1) eine Aminosäuresequenz umfasst, die zu der in SEQ ID NO:2 angegebenen Aminosäuresequenz über deren Gesamtlänge zu mindestens 80% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine der Deletionen D183* and G184* in der Zählung gemäß SEQ ID NO:2 aufweist; und/oder
d) die Amylase (A1) eine Aminosäuresequenz umfasst, die zu der in SEQ ID NO:2 angegebenen Aminosäuresequenz über deren Gesamtlänge zu mindestens 80% identisch ist und in der Zählung gemäß SEQ ID NO:2 folgende Aminosäuresubstitutionen und/oder Deletionen aufweist:
(i) M9L + M323T;
(ii) M9L + M202L/T/V/I + M323T;
(iii) M9L + N195F + M202L/T/V/I + M323T;
(iv) M9L + R118K + D183* + G184* + R320K + M323T + R458K;
(v) M9L + R118K + D183* + G184* + M202L/T/V/I + R320K + M323T + R458K;
(vi) M9L + G149A + G182T + G186A + M202L + T257I + Y295F + N299Y + M323T + A339S + E345R;
(vii) M9L + G149A + G182T + G186A + M202I + T257I + Y295F + N299Y + M323T + A339S + E345R;
(viii) M9L + R118K + G149A + G182T + D183* + G184* + G186A + M202L + T257I + Y295F + N299Y + R320K + M323T + A339S + E345R + R458K;
(ix) M9L + R118K + G149A + G182T + D183* + G184* + G186A + N195F + M202L + T257I + Y295F + N299Y + R320K + M323T + A339S + E345R + R458K;
(x) M9L + R118K + G149A + G182T + D183* + G184* + G186A + M202I + T257I + Y295F + N299Y + R320K + M323T + A339S + E345R + R458K;
(xi) M9L + R118K + D183* + D184* + N195F + M202L + R320K + M323T + R458K;
(xii) M9L + R118K + D183* + D184* + N195F + M202T + R320K + M323T + R458K;
(xiii) M9L + R118K + D183* + D184* + N195F + M202I + R320K + M323T + R458K;
(xiv) M9L + R118K + D183* + D184* + N195F + M202V + R320K + M323T + R458K;
(xv) M9L + R118K + N150H + D183* + D184* + N195F + M202L + V214T + R320K + M323T + R458K; oder
(xvi) M9L + R118K + D183* + D184* + N195F + M202L + V214T + R320K + M323T + E345N + R458K.

2. Reinigungsmittel nach Anspruch 1, **dadurch gekennzeichnet, dass** das Reinigungsmittel mindestens eine erste Amylase (A2) und mindestens eine zweite Amylase (A1) umfasst, wobei
(A) die erste Amylase (A2) in der festen Enzymformulierung enthalten ist; und
(B) die zweite Amylase (A1) in der flüssigen Enzymformulierung enthalten ist.

3. Reinigungsmittel nach Anspruch 1 oder 2, **dadurch gekennzeichnet, dass**
a) die Amylase (A2) eine α-Amylase aus *Bacillus sp.* No. 707 oder ein funktionales Fragment oder eine Variante davon ist; und/oder
b) die Amylase (A2) eine Aminosäuresequenz umfasst, die zu der in SEQ ID NO:1 angegebenen Aminosäuresequenz über deren Gesamtlänge zu mindestens 80% identisch ist und optional mindestens eine Aminosäuresubstitution an einer der Positionen 172, 202, 208, 255 und 261 in der Zählung gemäß SEQ ID NO:1 aufweist, insbesondere ausgewählt aus der Gruppe bestehend aus M202L, M202V, M202S, M202T, M202I, M202Q, M202W, S255N und R172Q.

4. Reinigungsmittel nach einem der Ansprüche 1 bis 3, **dadurch gekennzeichnet, dass** das Reinigungsmittel mindestens eine erste Protease (P2) und mindestens eine zweite Protease (P1) enthält, wobei
(A) die erste Protease (P2) in der festen Enzymformulierung enthalten ist; und
(B) die zweite Protease (P1) in der flüssigen Enzymformulierung enthalten ist.

5. Reinigungsmittel nach einem der Ansprüche 1 bis 4, **dadurch gekennzeichnet, dass** die Protease (P2) eine Protease aus *Bacillus alkalophilus* PB92 oder ein funktionales Fragment oder eine Variante davon umfasst, insbesondere eine, die eine Aminosäuresequenz aufweist, die zu der in SEQ ID NO:4 angegebenen Aminosäuresequenz über deren Gesamtlänge zu mindestens 80%, bevorzugt zu mindestens 90%, insbesondere zu 100% identisch ist und die optional mindestens eine Aminosäuresubstitution an einer der folgenden Positionen 32, 33, 48-54, 58-62, 94-107, 116, 123-133, 150, 152-156, 158-161, 164, 169, 175-186, 197, 198, 203-216 in der Zählung gemäß SEQ ID NO:4 aufweist, insbesondere mindestens eine Aminosäuresubstitution an einer, zwei, drei oder vier der folgenden Positionen 116, 126, 127, 128 und 160 in der Zählung gemäß SEQ ID NO:4, vorzugsweise mindestens eine, noch bevorzugter mehrere, am bevorzugtesten jede der Aminosäuresubstitution G116V, S126L, P127Q und/oder S128A.

6. Reinigungsmittel nach einem der Ansprüche 1 bis 5, **dadurch gekennzeichnet, dass** die Protease (P1)
(a) ein Subtilisin 309 aus *Bacillus lentus* oder ein funktionales Fragment oder eine Variante davon umfasst, insbesondere eine, die eine Aminosäuresequenz umfasst, die zu der in SEQ ID NO:3 angegebenen Aminosäuresequenz über deren Gesamtlänge zu mindestens 80%, bevorzugt zu mindestens 90%, insbesondere zu 100% identisch ist und mindestens eine Aminosäuresubstitution an einer der Positionen 9, 15, 66, 212 und 239 in der Zählung gemäß SEQ ID NO:3 aufweist, vorzugsweise ausgewählt aus der Gruppe bestehend aus S9R, A15T, V66A, N212D und Q239R;
(b) ein Subtilisin 309 aus *Bacillus lentus* oder ein funktionales Fragment oder eine Variante davon umfasst, insbesondere eine, die eine Aminosäuresequenz umfasst, die zu der in SEQ ID NO:3 angegebenen Aminosäuresequenz über deren Gesamtlänge zu mindestens 80% bevorzugt zu mindestens 90%, insbesondere zu 100% identisch ist und eine Aminosäuresubstitution an der Position 99 und eine Insertion von einer Aminosäure zwischen den Aminosäuren an Positionen 99 und 100 in der Zählung gemäß SEQ ID NO:3 aufweist, vorzugsweise ausgewählt aus S99A und/oder S99_G100InsD;
(c) eine alkalische Protease aus *Bacillus lentus* DSM 5483 oder ein funktionales Fragment oder eine Variante davon umfasst, insbesondere eine, die eine Aminosäuresequenz aufweist, die zu der in SEQ ID NO:5 angegebenen Aminosäuresequenz über deren Gesamtlänge zu mindestens 80%, bevorzugt zu mindestens 90%, insbesondere zu 100% identisch ist und die optional mindestens eine Aminosäuresubstitution an einer, zwei, drei oder vier der folgenden Positionen 3, 4, 99 und 199 in der Zählung gemäß SEQ ID NO:5 aufweist, insbesondere die Aminosäuresubstitution R99E oder R99D, sowie optional zusätzlich mindestens eine oder zwei, vorzugsweise alle drei der Aminosäuresubstitutionen S3T, V4I und V199I; oder
(d) eine Aminosäuresequenz gemäß einer der SEQ ID NOs: 6-10 aufweist.

7. Reinigungsmittel nach einem der Ansprüche 1 bis 6, **dadurch gekennzeichnet, dass**
(A) die feste Enzymformulierung in einer Menge von 0,01 bis 5 Gew.-%, vorzugsweise 0,2 bis 2 Gew.-%, bezogen auf das Gesamtgewicht des Reinigungsmittels enthalten ist, wobei die feste Enzymformulierung insbesondere einen Aktivenzymgehalt von 2 bis 20 Gew.-% aufweist; und/oder
(B) die flüssige Enzymformulierung in einer Menge von 0,01 bis 8 Gew.-%, vorzugsweise 0,3 bis 6 Gew.-%, bezogen auf das Gesamtgewicht des Reinigungsmittels enthalten ist, wobei die flüssige Enzymformulierung insbesondere einen Aktivenzymgehalt von 1 bis 6 Gew.-% aufweist.

8. Reinigungsmittel nach einem der Ansprüche 1 bis 7, **dadurch gekennzeichnet, dass** das Reinigungsmittel die flüssige Enzymformulierung und die feste Enzymformulierung in einem Massenverhältnis von 10:1 bis 1:10, vorzugsweise 1:1, enthält.

9. Reinigungsmittel nach einem der Ansprüche 1 bis 8, **dadurch gekennzeichnet, dass** das Reinigungsmittel
(A) mindestens eine phosphathaltige Builderkomponente, vorzugsweise ein Polyphosphat, insbesondere Kalium- und/oder Natriumtripolyphosphat, und/oder mindestens eine nicht-phosphathaltige Builderkomponente oder Co-Builder, insbesondere Citrat und/oder Phosphonat, vorzugsweise in Form des Natriumsalzes, enthält;
(B) mindestens einen mehrwertigen Alkohol, insbesondere ausgewählt aus Glycerin und Mischungen von Glycerin und 1,2-Propylenglykol enthält;
(C) einen Wassergehalt kleiner 50, vorzugsweise kleiner 25 Gew.-% aufweist,

10. Reinigungsmittel nach einem der Ansprüche 1 bis 9 **dadurch gekennzeichnet, dass** das Reinigungsmittel mindestens ein Sulfopolymer enthält.

11. Reinigungsmittel nach einem der Ansprüche 1 bis 10, **dadurch gekennzeichnet, dass** das Reinigungsmittel ferner einen oder mehrere der Stoffe ausgewählt aus der Gruppe bestehend aus Komplexbildnern, pH-Stellmitteln, nichtionischen, anionischen, kationischen und amphoteren Tensiden, Bleichmitteln, Bleichaktivatoren, Bleichkatalysatoren, Polymeren, Nicht-Protease- und Nicht-Amylase-Enzymen, Verdickern, Sequestrierungsmitteln, Elektrolyten, Korrosionsinhibitoren, insbesondere Silberschutzmitteln, Glaskorrosionsinhibitoren, Schauminhibitoren, Farbstoffen, Duftstoffen, Bitterstoffen, und antimikrobiellen Wirkstoffen enthält.

12. Reinigungsmittel nach einem der Ansprüche 1 bis 11, **dadurch gekennzeichnet, dass** das Reinigungsmittel ein maschinelles Geschirrspülmittel ist und
(1) in vorportionierter Form vorliegt; und/oder
(2) mehrere räumlich voneinander getrennte Zusammensetzungen aufweist; und/oder
(3) sich in einer wasserunlöslichen, wasserlöslichen oder wasserdispergierbaren Verpackung befindet, insbesondere in einer Polyvinylalkohol-haltigen Folie.

13. Verwendung des Reinigungsmittels nach einem der Ansprüche 1 bis 12 als Geschirrspülmittel, insbesondere maschinelles Geschirrspülmittel.

14. Verfahren zur Reinigung von Geschirr in einer automatischen Geschirrspülmaschine, **dadurch gekennzeichnet, dass** ein Reinigungsmittel nach einem der Ansprüche 1 bis 12 eingesetzt und vorzugsweise während des Durchlaufens eines Geschirrspülprogramms vor Beginn des Hauptspülgangs oder im Verlaufe des Hauptspülgangs in den Innenraum der Geschirrspülmaschine eindosiert wird.

## Claims

1. A liquid cleaning agent, in particular an automatic dishwasher detergent, containing
(1) at least one liquid enzyme formulation which contains at least one amylase (A1) and optionally at least one protease (P1); and
(2) at least one solid enzyme formulation, preferably in the form of granular material, which contains at least one protease (P2) and/or at least one amylase (A2), the solid enzyme formulation being suspended homogeneously in the liquid dishwasher detergent,
**characterized in that**
a) the amylase (A1) is an AA560 α-amylase from *Bacillus sp.* or a functional fragment thereof; and/or
b) the amylase (A1) has an amino acid sequence which is at least 80% identical to the amino acid sequence specified in SEQ ID NO:2 over the entire length thereof and optionally has at least one amino acid substitution at one of the positions 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 and 484 and/or one of the deletions D183* and G184* in the numbering according to SEQ ID NO:2; and/or
d) the amylase (A1) has an amino acid sequence which is at least 80% identical to the amino acid sequence specified in SEQ ID NO:2 over the entire length thereof and, in the numbering according to SEQ ID NO:2, has the following amino acid substitutions and/or deletions:
(i) M9L + M323T;
(ii) M9L + M202L/T/V/I + M323T;
(iii) M9L + N195F + M202L/T/V/I + M323T;
(iv) M9L + R118K + D183* + G184* + R320K + M323T + R458K;
(v) M9L + R118K + D183* + G184* + M202L/T/V/I + R320K + M323T + R458K;
(vi) M9L + G149A + G182T + G186A + M202L + T257I + Y295F + N299Y + M323T + A339S + E345R;
(vii) M9L + G149A + G182T + G186A + M202I + T257I + Y295F + N299Y + M323T + A339S + E345R;
(viii) M9L + R118K + G149A + G182T + D183* + G184* + G186A + M202L + T257I + Y295F + N299Y + R320K + M323T + A339S + E345R + R458K;
(ix) M9L + R118K + G149A + G182T + D183* + G184* + G186A + N195F + M202L + T257I + Y295F + N299Y + R320K + M323T + A339S + E345R + R458K;
(x) M9L + R118K + G149A + G182T + D183* + G184* + G186A + M202I + T257I + Y295F + N299Y + R320K + M323T + A339S + E345R + R458K;
(xi) M9L + R118K + D183* + D184* + N195F + M202L + R320K + M323T + R458K;
(xii) M9L + R118K + D183* + D184* + N195F + M202T + R320K + M323T + R458K;
(xiii) M9L + R118K + D183* + D184* + N195F + M202I + R320K + M323T + R458K;
(xiv) M9L + R118K + D183* + D184* + N195F + M202V + R320K + M323T + R458K;
(xv) M9L + R118K + N150H + D183* + D184* + N195F + M202L + V214T + R320K + M323T + R458K; or
(xvi) M9L + R118K + D183* + D184* + N195F + M202L + V214T + R320K + M323T + E345N + R458K.

2. The cleaning agent according to claim 1, **characterized in that** the cleaning agent comprises at least one first amylase (A2) and at least one second amylase (A1),
(A) the first amylase (A2) being contained in the solid enzyme formulation; and
(B) the second amylase (A1) being contained in the liquid enzyme formulation.

3. The cleaning agent according to claim 1 or 2, **characterized in that**
a) the amylase (A2) is an α-amylase from *Bacillus sp.* No. 707 or a functional fragment or a variant thereof; and/or
b) the amylase (A2) has an amino acid sequence which is at least 80% identical to the amino acid sequence specified in SEQ ID NO:1 over the entire length thereof, and optionally has at least one amino acid substitution at one of the positions 172, 202, 208, 255 and 261 in the numbering according to SEQ ID NO:1, in particular selected from the group consisting of M202L, M202V, M202S, M202T, M202I, M202Q, M202W, S255N and R172Q.

4. The cleaning agent according to one of claims 1 to 3,
**characterized in that** the cleaning agent contains at least one first protease (P2) and at least one second protease (P1),
(A) the first protease (P2) being contained in the solid enzyme formulation; and
(B) the second protease (P1) being contained in the liquid enzyme formulation.

5. The cleaning agent according to one of claims 1 to 4,
**characterized in that** the protease (P2) comprises a protease from *Bacillus alkalophilus* PB92 or a functional fragment or a variant thereof, in particular one which has an amino acid sequence which is at least 80%, preferably at least 90%, in particular 100%, identical to the amino acid sequence specified in SEQ ID NO:4 over the entire length thereof and optionally has at least one amino acid substitution at one of the following positions 32, 33, 48-54, 58-62, 94-107, 116, 123-133, 150, 152-156, 158-161, 164, 169, 175-186, 197, 198, 203-216 in the numbering according to SEQ ID NO:4, in particular at least one amino acid substitution at one, two, three or four of the following positions 116, 126, 127, 128 and 160 in the numbering according to SEQ ID NO:4, preferably at least one, even more preferably a plurality, most preferably each, of the amino acid substitutions G116V, S126L, P127Q and/or S128A.

6. The cleaning agent according to one of claims 1 to 5,
**characterized in that** the protease (P1)
(a) comprises a subtilisin 309 from *Bacillus lentus* or a functional fragment or a variant thereof, in particular one which has an amino acid sequence which is at least 80%, preferably at least 90%, in particular 100%, identical to the amino acid sequence specified in SEQ ID NO:3 over the entire length thereof and has at least one amino acid substitution at one of the positions 9, 15, 66, 212 and 239 in the numbering according to SEQ ID NO:3, preferably selected from the group consisting of S9R, A15T, V66A, N212D and Q239R;
(b) comprises a subtilisin 309 from *Bacillus lentus* or a functional fragment or a variant thereof, in particular one which has an amino acid sequence which is at least 80%, preferably at least 90%, in particular 100%, identical to the amino acid sequence specified in SEQ ID NO:3 over the entire length thereof and has an amino acid substitution at the position 99 and an insertion of an amino acid between the amino acids at positions 99 and 100 in the numbering according to SEQ ID NO:3, preferably selected from S99A and/or S99_G100InsD;
(c) comprises an alkaline protease from *Bacillus lentus* DSM 5483 or a functional fragment or a variant thereof, in particular one which has an amino acid sequence which is at least 80%, preferably at least 90%, in particular 100%, identical to the amino acid sequence specified in SEQ ID NO:5 over the entire length thereof and optionally has at least one amino acid substitution at one, two, three or four of the following positions 3, 4, 99 and 199 in the numbering according to SEQ ID NO:5, in particular the amino acid substitution R99E or R99D, and optionally additionally at least one or two, preferably all three, of the amino acid substitutions S3T, V4I and V199I; or
(d) has an amino acid sequence according to one of SEQ ID NOs: 6-10.

7. The cleaning agent according to one of claims 1 to 6,
**characterized in that**
(A) the solid enzyme formulation is contained in an amount of 0.01 to 5 wt.%, preferably from 0.2 to 2 wt.%, based on the total weight of the cleaning agent, the solid enzyme formulation having in particular an active enzyme content of 2 to 20 wt. %; and/or
(B) the liquid enzyme formulation is contained in an amount of 0.01 to 8 wt.%, preferably from 0.3 to 6 wt.%, based on the total weight of the cleaning agent, the liquid enzyme formulation having in particular an active enzyme content of 1 to 6 wt.%.

8. The cleaning agent according to one of claims 1 to 7,
**characterized in that** the cleaning agent contains the liquid enzyme formulation and the solid enzyme formulation in a weight ratio of 10:1 to 1:10, preferably 1:1.

9. The cleaning agent according to one of claims 1 to 8,
**characterized in that** the cleaning agent
(A) contains at least one phosphate-containing builder component, preferably a polyphosphate, in particular potassium and/or sodium triphosphate, and/or at least one non-phosphate-containing builder component or cobuilder, in particular citrate and/or phosphonate, preferably in the form of the sodium salt;
(B) contains at least one polyhydric alcohol, in particular selected from glycerol and mixtures of glycerol and 1,2-propylene glycol;
(C) has a water content of less than 50 wt.%, preferably less than 25 wt.%,

10. The cleaning agent according to one of claims 1 to 9,
**characterized in that** the cleaning agent contains at least one sulfopolymer.

11. The cleaning agent according to one of claims 1 to 10,
**characterized in that** the cleaning agent further contains one or more of the substances selected from the group consisting of complexing agents, pH adjusters, nonionic, anionic, cationic and amphoteric surfactants, bleaching agents, bleach activators, bleach catalysts, polymers, non-protease and nonamylase enzymes, thickeners, sequestering agents, electrolytes, corrosion inhibitors, in particular silver protecting agents, glass corrosion inhibitors, foam inhibitors, dyes, fragrances, bitterns, and antimicrobial active ingredients.

12. The cleaning agent according to one of claims 1 to 11, **characterized in that** the cleaning agent is an automatic dishwasher detergent and
(1) is in a pre-portioned form; and/or
(2) has a plurality of compositions that are spatially separated from one another; and/or
(3) is in a water-insoluble, water-soluble or water-dispersible packaging, in particular in a film containing polyvinyl alcohol.

13. A use of the cleaning agent according to one of claims 1 to 12 as a dishwasher detergent, in particular as an automatic dishwasher detergent.

14. A method for cleaning dishes in an automatic dishwasher,
**characterized in that** a cleaning agent according to one of claims 1 to 12 is used and is dispensed into the interior of the dishwasher preferably while a dishwashing program is running, before the main washing cycle begins or during the main washing cycle.

## Revendications

1. Agent de nettoyage liquide, en particulier agent de lavage de vaisselle en machine, contenant
(1) au moins une formulation enzymatique liquide qui contient au moins une amylase (A1) et éventuellement au moins une protéase (P1) ; et
(2) au moins une formulation enzymatique solide, de préférence sous forme de granulés, qui contient au moins une protéase (P2) et/ou au moins une amylase (A2), dans lequel la formulation enzymatique solide est en suspension de manière homogène dans l'agent de lavage de vaisselle liquide,
**caractérisé en ce que**
a) l'amylase (A1) est une α-amylase AA560 de *Bacillus sp.* ou un fragment fonctionnel de celle-ci ; et/ou
b) l'amylase (A1) comprend une séquence d'acides aminés qui est identique sur toute sa longueur à au moins 80 % à la séquence d'acides aminés indiquée dans SEQ ID NO : 2 et éventuellement au moins une substitution d'acide aminé à l'une des positions 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 et 484 et/ou l'une des délétions D183* et G184* dans le comptage conformément à SEQ ID NO : 2 ; et/ou
d) l'amylase (A1) comprend une séquence d'acides aminés qui est identique sur toute sa longueur à au moins 80 % à la séquence d'acides aminés indiquée dans SEQ ID NO : 2 et qui présente les substitutions d'acides aminés et/ou les délétions suivantes dans le comptage conformément à SEQ ID NO : 2 :
(i) M9L + M323T ;
(ii) M9L + M202L/T/V/I + M323T ;
(iii) M9L + N195F + M202L/T/V/I + M323T ;
(iv) M9L + R118K + D183* + G184* + R320K + M323T + R458K ;
(v) M9L + R118K + D183* + G184* + M202L/T/V/I + R320K + M323T + R458K ;
(vi) M9L + G149A + G182T + G186A + M202L + T257I + Y295F + N299Y + M323T + A339S + E345R ;
(vii) M9L + G149A + G182T + G186A + M202I + T257I + Y295F + N299Y + M323T + A339S + E345R ;
(viii) M9L + R118K + G149A + G182T + D183* + G184* + G186A + M202L + T257I + Y295F + N299Y + R320K + M323T + A339S + E345R + R458K ;
(ix) M9L + R118K + G149A + G182T + D183* + G184* + G186A + N195F + M202L + T257I + Y295F + N299Y + R320K + M323T + A339S + E345R + R458K ;
(x) M9L + R118K + G149A + G182T + D183* + G184* + G186A + M202I + T257I + Y295F + N299Y + R320K + M323T + A339S + E345R + R458K ;
(xi) M9L + R118K + D183* + D184* + N195F + M202L + R320K + M323T + R458K ;
(xii) M9L + R118K + D183* + D184* + N195F + M202T + R320K + M323T + R458K ;
(xiii) M9L + R118K + D183* + D184* + N195F + M202I + R320K + M323T + R458K ;
(xiv) M9L + R118K + D183* + D184* + N195F + M202V + R320K + M323T + R458K ;
(xv) M9L + R118K + N150H + D183* + D184* + N195F + M202L + V214T + R320K + M323T + R458K ; ou
(xvi) M9L + R118K + D183* + D184* + N195F + M202L + V214T + R320K + M323T + E345N + R458K.

2. Agent de nettoyage selon la revendication 1, **caractérisé en ce que** l'agent de nettoyage comprend au moins une première amylase (A2) et au moins une seconde amylase (A1), dans lequel
(A) la première amylase (A2) est contenue dans la formulation enzymatique solide ; et
(B) la seconde amylase (A1) est contenue dans la formulation enzymatique liquide.

3. Agent de nettoyage selon la revendication 1 ou 2, **caractérisé en ce que**
a) l'amylase (A2) est une α-amylase de *Bacillus sp.* n° 707 ou un fragment fonctionnel ou une variante de celle-ci ; et/ou
b) l'amylase (A2) comprend une séquence d'acides aminés qui est identique sur toute sa longueur à au moins 80 % à la séquence d'acides aminés indiquée dans SEQ ID NO : 1 et présente éventuellement au moins une substitution d'acide aminé à l'une des positions 172, 202, 208, 255 et 261 dans le comptage conformément à SEQ ID NO : 1, en particulier choisie dans le groupe constitué de M202L, M202V, M202S, M202T, M202I, M202Q, M202W, S255N et R172Q.

4. Agent de nettoyage selon l'une des revendications 1 à 3,
**caractérisé en ce que** l'agent de nettoyage contient au moins une première protéase (P2) et au moins une seconde protéase (P1), dans lequel
(A) la première protéase (P2) est contenue dans la formulation enzymatique solide ; et
(B) la seconde protéase (P1) est contenue dans la formulation enzymatique liquide.

5. Agent de nettoyage selon l'une des revendications 1 à 4,
**caractérisé en ce que** la protéase (P2) comprend une protéase de *Bacillus alkalophilus* PB92 ou un fragment fonctionnel ou une variante de celle-ci, en particulier une protéase ou un fragment fonctionnel ou une variante de celle-ci qui présente une séquence d'acides aminés qui est identique sur toute sa longueur à au moins 80 %, de préférence à au moins 90 %, en particulier à au moins 100 % à la séquence d'acides aminés indiquée dans SEQ ID NO : 4, et qui présente éventuellement au moins une substitution d'acide aminé à l'une des positions suivantes 32, 33, 48 à 54, 58 à 62, 94 à 107, 116, 123 à 133, 150, 152 à 156, 158 à 161, 164, 169, 175 à 186, 197, 198, 203 à 216 dans le comptage conformément à SEQ ID NO : 4, en particulier au moins une substitution d'acide aminé à une, deux, trois ou quatre des positions suivantes 116, 126, 127, 128 et 160 dans le comptage conformément à SEQ ID NO : 4, de préférence au moins une substitution d'acide aminé, de manière encore plus préférée plusieurs substitutions d'acides aminés, de manière la plus préférée chacune des substitutions d'acides aminés parmi G116V, S126L, P127Q et/ou S128A.

6. Agent de nettoyage selon l'une des revendications 1 à 5,
**caractérisé en ce que** la protéase (P1)
(a) comprend une subtilisine 309 de *Bacillus lentus* ou un fragment fonctionnel ou une variante de celle-ci, en particulier une subtilisine ou un fragment fonctionnel ou une variante de celle-ci qui présente une séquence d'acides aminés qui est identique sur toute sa longueur à au moins 80 %, de préférence à au moins 90 %, en particulier à au moins 100 % à la séquence d'acides aminés indiquée dans SEQ ID NO : 3, et qui présente au moins une substitution d'acide aminé à l'une des positions 9, 15, 66, 212 et 239 dans le comptage conformément à SEQ ID NO : 3, de préférence choisie dans le groupe constitué de S9R, A15T, V66A, N212D et Q239R ;
(b) comprend une subtilisine 309 de *Bacillus lentus* ou un fragment fonctionnel ou une variante de celle-ci, en particulier une subtilisine ou un fragment fonctionnel ou une variante de celle-ci qui présente une séquence d'acides aminés qui est identique sur toute sa longueur à au moins 80 %, de préférence à au moins 90 %, en particulier à au moins 100 % à la séquence d'acides aminés indiquée dans SEQ ID NO : 3, et qui présente une substitution d'acide aminé à la position 99 et une insertion d'un acide aminé entre les acides aminés aux positions 99 et 100 dans le comptage conformément à SEQ ID NO : 3, de préférence choisie parmi S99A et/ou S99_G100InsD ;
(c) comprend une protéase alcaline de *Bacillus lentus* DSM 5483 ou un fragment fonctionnel ou une variante de celle-ci, en particulier une protéase alcaline ou un fragment fonctionnel ou une variante de celle-ci qui présente une séquence d'acides aminés qui est identique sur toute sa longueur à au moins 80 %, de préférence à au moins 90 %, en particulier à au moins 100 % à la séquence d'acides aminés indiquée dans SEQ ID NO : 5, et qui présente éventuellement au moins une substitution d'acide aminé à une, deux, trois ou quatre des positions suivantes 3, 4, 99 et 199 dans le comptage conformément à SEQ ID NO : 5, en particulier la substitution d'acide aminé R99E ou R99D, et éventuellement en outre au moins une ou deux, de préférence les trois substitutions d'acides aminés parmi S3T, V4I et V199I ; ou
(d) présente une séquence d'acides aminés conformément à l'une parmi les SEQ ID NO : 6 à 10.

7. Agent de nettoyage selon l'une des revendications 1 à 6,
**caractérisé en ce que**
(A) la formulation enzymatique solide est contenue en une quantité allant de 0,01 à 5 % en poids, de préférence de 0,2 à 2 % en poids, par rapport au poids total de l'agent de nettoyage, dans lequel la formulation enzymatique solide présente en particulier une teneur en enzymes actives allant de 2 à 20 % en poids ; et/ou
(B) la formulation enzymatique liquide est contenue en une quantité allant de 0,01 à 8 % en poids, de préférence de 0,3 à 6 % en poids, par rapport au poids total de l'agent de nettoyage, dans lequel la formulation enzymatique liquide présente en particulier une teneur en enzymes actives allant de 1 à 6 % en poids.

8. Agent de nettoyage selon l'une des revendications 1 à 7,
**caractérisé en ce que** l'agent de nettoyage contient la formulation enzymatique liquide et la formulation enzymatique solide dans un rapport massique allant de 10:1 à 1:10, de préférence de 1:1.

9. Agent de nettoyage selon l'une des revendications 1 à 8,
**caractérisé en ce que** l'agent de nettoyage
(A) contient au moins un composant adjuvant contenant du phosphate, de préférence un polyphosphate, en particulier du tripolyphosphate de potassium et/ou de sodium, et/ou au moins un composant adjuvant ou co-adjuvant ne contenant pas de phosphate, en particulier du citrate et/ou du phosphonate, de préférence sous la forme du sel de sodium ;
(B) contient au moins un polyalcool, en particulier choisi parmi glycérol et mélanges de glycérol et de 1,2-propylène glycol ;
(C) présente une teneur en eau inférieure à 50, de préférence inférieure à 25 % en poids,

10. Agent de nettoyage selon l'une des revendications 1 à 9
**caractérisé en ce que** l'agent de nettoyage contient au moins un sulfopolymère.

11. Agent de nettoyage selon l'une des revendications 1 à 10,
**caractérisé en ce que** l'agent de nettoyage comprend en outre une ou plusieurs des substances choisies dans le groupe constitué d'agents complexants, agents de régulation du pH, tensioactifs non ioniques, anioniques, cationiques et amphotères, agents de blanchiment, activateurs de blanchiment, catalyseurs de blanchiment, polymères, enzymes non-protéases et non-amylases, épaississants, agents séquestrants, électrolytes, inhibiteurs de corrosion, en particulier agents de protection de l'argent, inhibiteurs de corrosion du verre, inhibiteurs de mousse, colorants, parfums, substances amères, et principes actifs antimicrobiens.

12. Agent de nettoyage selon l'une des revendications 1 à 11,
**caractérisé en ce que** l'agent de nettoyage est un agent de lavage de vaisselle en machine et
(1) se présente sous forme préportionnée ; et/ou
(2) présente plusieurs compositions séparées spatialement les unes des autres ; et/ou
(3) se trouve dans un emballage insoluble dans l'eau, soluble dans l'eau ou dispersable dans l'eau, en particulier dans un film contenant de l'alcool polyvinylique.

13. Utilisation de l'agent de nettoyage selon l'une des revendications 1 à 12 en tant qu'agent de lavage de vaisselle, en particulier en tant qu'agent de lavage de vaisselle en machine.

14. Procédé pour le nettoyage de vaisselle dans un lave-vaisselle automatique, **caractérisé en ce qu'un** agent de nettoyage selon l'une des revendications 1 à 12 est utilisé et de préférence dosé dans l'espace intérieur du lave-vaisselle pendant le fonctionnement d'un programme de lavage de vaisselle avant le début du cycle de lavage principal ou au cours du cycle de lavage principal.
